(19) Europäisches Patentamt
European Patent Office
Office européen des brevets

(11) **EP 3 936 118 A1**

(12) **EUROPEAN PATENT APPLICATION**
published in accordance with Art. 153(4) EPC

(43) Date of publication:
**12.01.2022 Bulletin 2022/02**

(21) Application number: **20759493.8**

(22) Date of filing: **24.02.2020**

(51) Int Cl.:
*A61K 9/14* (2006.01)          *A61K 9/16* (2006.01)
*A61K 31/7105* (2006.01)          *A61P 37/00* (2006.01)
*A61P 35/00* (2006.01)

(86) International application number:
**PCT/KR2020/002648**

(87) International publication number:
**WO 2020/171680 (27.08.2020 Gazette 2020/35)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **22.02.2019 US 201962808974 P**

(71) Applicant: Lemonex Inc.
Seoul 08826 (KR)

(72) Inventors:
• **WON, Cheol Hee**
  **Seoul 08741 (KR)**
• **KIM, Jung Ho**
  **Seoul 02595 (KR)**
• **KIM, Jun**
  **Seoul 08799 (KR)**

(74) Representative: **dompatent von Kreisler Selting Werner -
Partnerschaft von Patent- und Rechtsanwälten mbB
Deichmannhaus am Dom
Bahnhofsvorplatz 1
50667 Köln (DE)**

(54) **PHARMACEUTICAL COMPOSITION FOR IMMUNE ACTIVITY OR FOR PREVENTING OR TREATING CANCER**

(57) The present invention relates to a pharmaceutical composition for improving immune activity and a pharmaceutical composition for prevention or treatment of cancer, the pharmaceutical composition including: a blunt-ended hairpin RNA; and porous silica particles carrying the RNA in pores thereof, wherein the porous silica particles have an average pore diameter of 7 to 25 nm, and the inside of the pores is positively charged, thus to sufficiently support the RNA and stably deliver the supported RNA into the body.

[FIG. 1]

EP 3 936 118 A1

Printed by Jouve, 75001 PARIS (FR)

<110>    LEMONEX INC

<120>    COMPOSITION FOR IMMUNE ENHANCING OR PREVENTING OR TREATING OF CANCER

<130>    20P02035

<150>    US 62/808974
<151>    2019-02-22

<160>    25

<170>    KoPatentIn 3.0

<210>    1
<211>    32
<212>    RNA
<213>    Artificial Sequence

<220>
<223>    RIG I agonist


<400>    1
ggaucgaucg aucguucgcg aucgaucgau cc                                    32


<210>    2
<211>    32
<212>    RNA
<213>    Artificial Sequence

<220>
<223>    RIG I agonist


<400>    2
cgacgucgac gucguucgcg acgucgacgu cg                                    32


<210>    3
<211>    32
<212>    RNA
<213>    Artificial Sequence

<220>
<223>    RIG I agonist


<400>    3
gcacgucgac gugcuucggc acgucgacgu gc                                    32


<210>    4
<211>    32
<212>    RNA
<213>    Artificial Sequence

<220>
<223>    RIG I agonist


<400>    4

ggacgucgac guccuucggg acgucgacgu cc                                    32


<210>    5
<211>    32
<212>    RNA
<213>    Artificial Sequence

<220>
<223>    RIG I agonist


<400>    5
cgaucgaucg aucguucgcg aucgaucgau cg                                    32


<210>    6
<211>    32
<212>    RNA
<213>    Artificial Sequence

<220>
<223>    RIG I agonist


<400>    6
ccaucgaucg aucguucgcg aucgaucgau gg                                    32


<210>    7
<211>    32
<212>    RNA
<213>    Artificial Sequence

<220>
<223>    RIG I agonist


<400>    7
ggaagcuacg auccuucggg aucguagcuu cc                                    32


<210>    8
<211>    32
<212>    RNA
<213>    Artificial Sequence

<220>
<223>    RIG I agonist


<400>    8
ggauccuugc uagguucgcc uagcaaggau cc                                    32


<210>    9
<211>    32
<212>    RNA
<213>    Artificial Sequence

<220>
<223>    RIG I agonist

```
<400>    9
ccaugguugc aagguucgcc uugcaaccau gg                                          32


<210>    10
<211>    34
<212>    RNA
<213>    Artificial Sequence

<220>
<223>    RIG I agonist


<400>    10
gcgauaggca uugccuucgg gcaaugccua ucgc                                        34


<210>    11
<211>    34
<212>    RNA
<213>    Artificial Sequence

<220>
<223>    RIG I agonist


<400>    11
gggaaagggu uucccuucgg ggaaacccuu uccc                                        34


<210>    12
<211>    40
<212>    RNA
<213>    Artificial Sequence

<220>
<223>    RIG I agonist


<400>    12
ccaaggaucg uaggaaccuu cggguuccua cgauccuugg                                  40


<210>    13
<211>    46
<212>    RNA
<213>    Artificial Sequence

<220>
<223>    RIG I agonist


<400>    13
gcgaaugcgu auccgaaugc guucgcgcau ucggauacgc auucgc                           46


<210>    14
<211>    46
<212>    RNA
<213>    Artificial Sequence

<220>
```

<223>    RIG I agonist


<400>    14
gcguaucgca auggguuucg cuucggcgaa acccauugcg auacgc                                    46


<210>    15
<211>    54
<212>    RNA
<213>    Artificial Sequence

<220>
<223>    RIG I agonist


<400>    15
cgcuuagcua ccgaaagcgu uucgcuucgg cgaaacgcuu ucgguagcua agcg          54


<210>    16
<211>    14
<212>    RNA
<213>    Artificial Sequence

<220>
<223>    RIG I agonist


<400>    16
gcagguucgc cugc                                    14


<210>    17
<211>    16
<212>    RNA
<213>    Artificial Sequence

<220>
<223>    RIG I agonist


<400>    17
cgaugcuucg cgaucg                                    16


<210>    18
<211>    20
<212>    RNA
<213>    Artificial Sequence

<220>
<223>    RIG I agonist


<400>    18
gcuuaggcuu cggccuaagc                                    20


<210>    19
<211>    24
<212>    RNA
<213>    Artificial Sequence

```
<220>
<223>    RIG I agonist


<400>    19
cgaagcuucg uucgcgaagc uucg                                          24


<210>    20
<211>    26
<212>    RNA
<213>    Artificial Sequence

<220>
<223>    RIG I agonist


<400>    20
cguaagcuuc guucgcgaag cuuacg                                        26


<210>    21
<211>    28
<212>    RNA
<213>    Artificial Sequence

<220>
<223>    RIG I agonist


<400>    21
ccgauagcuu cguucgcgaa gcuaucgg                                      28


<210>    22
<211>    30
<212>    RNA
<213>    Artificial Sequence

<220>
<223>    RIG I agonist


<400>    22
ccguaaggcu ucguucgcga agccuuacgg                                    30


<210>    23
<211>    58
<212>    RNA
<213>    Artificial Sequence

<220>
<223>    RIG I agonist


<400>    23
gcgauacgcu uugcguaacg cuuugcguuc gcgcaaagcg uuacgcaaag cguaucgc     58


<210>    24
<211>    60
```

```
<212>      RNA
<213>      Artificial Sequence

<220>
<223>      RIG I agonist


<400>      24
cgcguaagcc uaugcgaauc gguaugcguu cgcgcauagc cauucgcaua ggcuuacgcg      60

                                                                       60


<210>      25
<211>      62
<212>      RNA
<213>      Artificial Sequence

<220>
<223>      RIG I agonist


<400>      25
cgcgauaucg cauuggcaua cgcauugcgu ucgcgcaaug cguaugccaa ugcgauaucg      60

cg                                                                     62
```

**Description**

[Technical Field]

The present invention relates to a pharmaceutical composition for immune activity or for prevention or treatment of cancer.

[Background Art]

Standard vaccine strategies based on the induction of antibody-based immune responses have eradicated or nearly eradicated a number of previously fatal infectious diseases such as smallpox, polio and tetanus. However, these traditional human vaccines have not been effective or safe for use in other infectious diseases such as HIV and hepatitis, and non-infectious diseases such as cancer.

Next-generation immunotherapeutic products aimed at inducing cellular immune responses may overcome the limitations of traditional vaccines by recognizing and killing cancer cells and infected cells instead of the pathogen itself. Nucleic acid vaccines, and in particular, viral vectors have shown great potential for translation into clinic.

Cancer cells and many infectious agents have an ability to evade the immune system, which makes it difficult to create an effective vaccine. Classical vaccines often require adjuvants, for example, aluminum salts for optimal effectiveness, but conventional adjuvants are typically poor enhancers of cellular immune responses. Several strategies have been proposed to improve the quality and extent of cellular immune responses induced by viral vectors. A new type of genetic adjuvants has been developed to improve the cellular immune response induced by vector-based immunotherapy. Genetic adjuvants consist of a DNA sequence encoding an immune regulatory molecule.

Stone et al. (WO 2014/039961) disclose the use of a genetic adjuvant to induce the secretion of interferon alpha and beta to induce the expression of interferon-stimulated genes. In this approach, the nucleic acid vaccine encodes a fusion protein including a transmembrane portion of LMP1 protein in which the intracytoplasmic domain is replaced with an immune effector or adapter protein, such as the IPS1 protein, in addition to a transgene optionally encoding a marker protein or antigen. Activation of IFN-$\beta$ promoter stimulator (IPS1; also referred to as MAVS, VISA or Cardif) produces a strong T cell response through a STING (stimulator of the interferon gene) pathway. When expressed in cells, the transmembrane domain of LMP1 activates the STING pathway by spontaneously forming clusters that allow IPS1 to aggregate into intracytoplasmic clusters. The transmembrane domain of LMP1 fused with full-length murine IPS1 has been exhibited to induce the secretion of IFN-$\alpha$, IFN-$\beta$ and IL-6, and also induce the expression of maturation markers (CD40 and CCR7) and activation markers (CD80 and CD86) in mouse macrophages.

[Summary of Invention]

[Problems to be Solved by Invention]

An object of the present invention is to provide a pharmaceutical composition for improving immune activity.

Another object of the present invention is to provide a pharmaceutical composition for prevention or treatment of cancer.

[Means for Solving Problems]

To achieve the above objects, the following technical solutions are adopted in the present invention.

1. A pharmaceutical composition for improving immune activity, including: a blunt-ended hairpin RNA represented by Formula (1) below; and porous silica particles carrying the RNA in pores thereof,

wherein the porous silica particles have an average pore diameter of 7 to 25 nm, and the inside of the pores is positively charged,

[Formula 1]    5'-$P_a$-((N1-X1)$_b$-N2)-UUCG-(N3-(X2-N4)$_b$)-3'

(wherein P is a phosphoric acid group,
a is an integer of 2 to 5, and b is an integer of 1 to 5,
UUCG is a base to form a loop of the hairpin,
N1 and N2 are 2 to 4 bases selected from G or C, X1 and X2 are 1 to 5 bases selected from A or U, and the plurality of selected bases are the same as or different from one other,

N3 is complementarily bound with N2, X2 is complementarily bound with X1, and N4 is complementarily bound with N1, and

each base repeating b times is the same as or different from one another).

2. The composition according to the above 1, wherein the RNA is represented by Formula 2 below:

[Formula 2]     5'-$P_a$-((N1-X1)$_b$-N2)-UUCG-(N3-(X2-N4)$_b$)-3'

(wherein P is a phosphoric acid group,

a and b are each an integer of 2 to 4,

N1 and N2 are 2 to 4 bases selected from G or C, X1 and X2 are 2 to 4 bases selected from A or U, and the plurality of selected bases are the same as or different from one other,

N3 is complementarily bound with N2, X2 is complementarily bound with X1, and N4 is complementarily bound with N1, and

each base repeating b times is the same as or different from one another).

3. The composition according to the above 1, wherein the RNA has a length of 14 to 100 nt.

4. The composition according to the above 1, wherein the RNA includes any one of SEQ ID NOs: 1 to 25, as well as 2 to 4 phosphoric acid groups bound to the 5'-terminal.

5. The composition according to the above 1, wherein a zeta potential of the porous silica particles carrying RNA in the pores thereof is 5 to 65 mV.

6. The composition according to the above 1, wherein the zeta potential of the porous silica particles carrying the RNA in the pores thereof is 35 mV or less.

7. The composition according to the above 1, wherein the zeta potential of the particle without carrying RNA is 10 to 70 mV.

8. The composition according to the above 1, wherein a weight ratio of the particles and the RNA is 1: 5 to 20.

9. The composition according to the above 1, wherein the particle has a plurality of pores, and the pores extend from a surface to an inside of the particle.

10. The composition according to the above 1, wherein a BET surface area of the particles is 280 to 680 m²/g, and a particle diameter is 50 to 500 nm.

11. The composition according to the above 1, wherein the porous silica particles have pores of small pore particles having an average pore diameter of less than 5 nm, and the pores are expanded to an average diameter of 7 to 25 nm.

12. A pharmaceutical composition for prevention or treatment of cancer, including: a blunt-ended hairpin RNA represented by Formula (1) below; and porous silica particles carrying the RNA in pores thereof,

wherein the porous silica particles have an average pore diameter of 7 to 25 nm, and the inside of the pores is positively charged,

[Formula 1]     5'-$P_a$-((N1-X1)$_b$-N2)-UUCG-(N3-(X2-N4)$_b$)-3'

(wherein P is a phosphoric acid group,
a is an integer of 2 to 5, and b is an integer of 1 to 5,
UUCG is a base to form a loop of the hairpin,
N1 and N2 are 2 to 4 bases selected from G or C, X1 and X2 are 1 to 5 bases selected from A or U, and the plurality of selected bases are the same as or different from one other,
N3 is complementarily bound with N2, X2 is complementarily bound with X1, and N4 is complementarily bound with N1, and
each base repeating b times is the same as or different from one another).

13. The composition according to the above 12, wherein the RNA is represented by Formula 2 below:

[Formula 2]     5'-$P_a$-((N1-X1)$_b$-N2)-UUCG-(N3-(X2-N4)$_b$)-3'

(wherein P is a phosphoric acid group,

a and b are each an integer of 2 to 4,
N1 and N2 are 2 to 4 bases selected from G or C, X1 and X2 are 2 to 4 bases selected from A or U, and the plurality of selected bases are the same as or different from one other,

N3 is complementarily bound with N2, X2 is complementarily bound with X1, and N4 is complementarily bound with N1, and

each base repeating b times is the same as or different from one another)

14. The composition according to the above 12, wherein the RNA has a length of 14 to 100 nt.

15. The composition according to the above 12, wherein RNA includes any one sequence of SEQ ID NOs: 1 to 25, and 2 to 4 phosphoric acid groups bound to the 5-terminal.

16. The composition according to the above 12, wherein a zeta potential of the porous silica particles carrying the RNA in the pores thereof is 5 to 65 mV.

17. The composition according to the above 12, wherein the zeta potential of the porous silica particles carrying the RNA in the pores thereof is 35 mV or less.

18. The composition according to the above 12, wherein the zeta potential of the particle without carrying RNA is 10 to 70 mV.

19. The composition according to the above 12, wherein a weight ratio of the particles and the RNA is 1: 5 to 20.

20. The composition according to the above 12, wherein the particle has a plurality of pores, and the pores extend from a surface to an inside of the particle.

21. The composition according to the above 12, wherein a BET surface area of the particle is 280 to 680 $m^2/g$, and a particle diameter is 50 to 500 nm.

22. The composition according to the above 12, wherein the porous silica particles have pores of small pore particles having an average pore diameter of less than 5 nm, and the pores are expanded to an average diameter of 7 to 25 nm.

23. The composition according to the above 12, wherein the cancer is any one of breast cancer, ovarian cancer, cervical cancer, prostate cancer, testicular cancer, penile cancer, genitourinary tract cancer, testicular tumor, esophageal cancer, laryngeal cancer, gastric cancer, gastrointestinal cancer, skin cancer, keratinocyte cell tumor, follicular carcinoma, melanoma, lung cancer, small cell lung carcinoma, non-small cell lung carcinoma (NSCLC), lung adenocarcinoma, squamous cell carcinoma of the lung, colon cancer, pancreatic cancer, thyroid cancer, papillary cancer, bladder cancer, liver cancer, bile duct cancer, kidney cancer, bone cancer, bone marrow disorder, lymphatic disorder, hair cell cancer, oral and pharyngeal (oral) cancer, lip cancer, tongue cancer, oral cancer, salivary gland cancer, pharyngeal cancer, small intestine cancer, colon cancer, rectal cancer, kidney cancer, prostate cancer, vulvar cancer, thyroid cancer, colorectal cancer, endometrial cancer, uterine cancer, brain cancer, central nervous system cancer, peritoneal cancer, hepatocellular carcinoma, head cancer, neck cancer, Hodgkin disease and leukemia.

[Advantageous Effects]

The composition of the present invention may stably deliver the supported RNA into the body and release the same to the target so as to activate RIG-I signaling pathway and promote the expression of factors such as IFN-$\alpha$, $\beta$, and Viperin, thereby exhibiting immune activation effects.

The composition of the present invention may exhibit excellent anticancer activity by activating an interferon signaling pathway and/or an interferon-independent cell apoptosis pathway.

The composition of the present invention may reduce phenomena such as a formation of vacuoles in the cytoplasm during delivery into the cells.

[Brief Description of Drawings]

FIG. 1 is micrographs of porous silica particles according to one embodiment of the present invention.

FIG. 2 is micrographs of porous silica particles according to one embodiment of the present invention.

FIG. 3 is micrographs of small pore particles obtained in a manufacturing process of the porous silica particles according to one embodiment of the present invention.

FIG. 4 is micrographs of the small pore particles according to one embodiment of the present invention.

FIG. 5 is micrographs of the porous silica particles for each pore diameter according to one embodiment of the present invention. DDV (Degradable Delivery Vehicle) is the particles according to an embodiment, wherein the number in parenthesis means the diameter of the particle and the number of subscripts means the pore diameter. For example, DDV $(200)_{10}$ refers to a particle having a particle diameter (that is, particle size) of 200 nm and a pore diameter of 10 nm according to an embodiment.

FIG. 6 is micrographs to identify biodegradability of the porous silica particles according to one embodiment of the present invention.

FIG. 7 a view illustrating a tube having a cylindrical permeable membrane according to one illustrative example.

FIG. 8 is a graph illustrating results of decreasing absorbance of the porous silica particles over time according to one embodiment of the present invention.

FIG. 9 is diagrams illustrating results of decreasing absorbance of the porous silica particles for each particle size over time according to one embodiment of the present invention.

FIG. 10 is diagrams illustrating results of decreasing absorbance of the porous silica particles for each pore diameter over time according to one embodiment of the present invention.

FIG. 11 is a graph illustrating results of decreasing absorbance of the porous silica particles for each pH of the environment over time according to one embodiment of the present invention.

FIG. 12 is a graph illustrating results of decreasing absorbance of the porous silica particles over time according to one embodiment of the present invention.

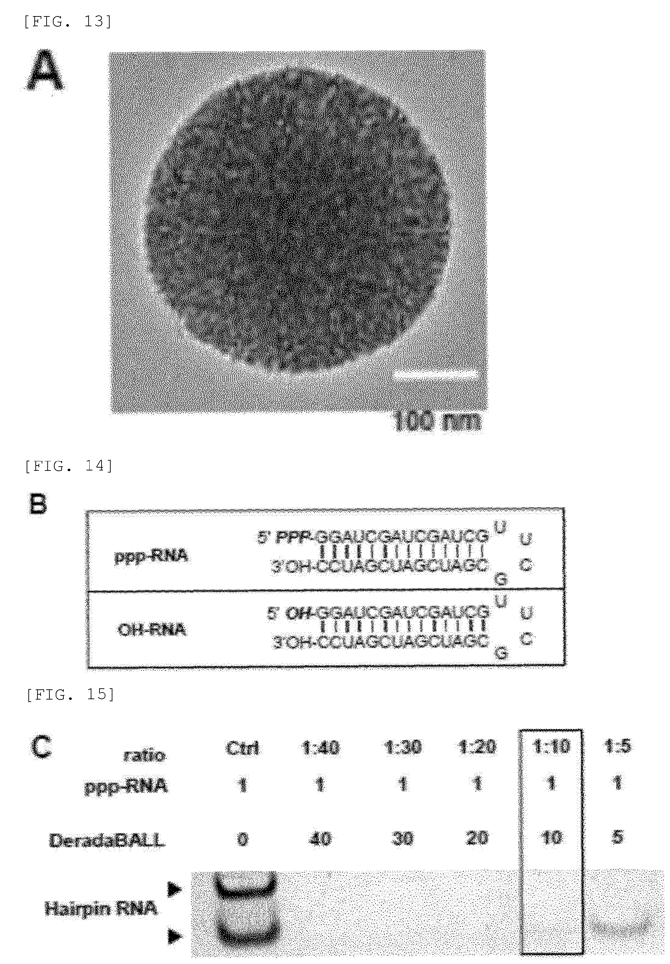

FIGS. 13 to 17 are diagrams illustrating analyzed results of characteristics of porous silica particles (LEM-S403) carrying RNA according to an embodiment of the present invention, wherein A shows DegradaBALL's electron microscope image and physical properties (surface area, micropore size, surface charge and average particle size); B shows sequence and structure of synthetic RIG-I ligand (5'-triphosphate hairpin RNA, ppp-RNA) and control hairpin RNA (OH-RNA); C shows SDS-PAGE analysis image of the supernatant which was obtained by mixing DegradaBALL and ppp-RNA at various weight ratios, followed by centrifugation; D shows real-time high-resolution fluorescence images wherein OH-RNA was released from the inside of A549 cells when A549 cells were treated with DegradaBALL carrying fluorescently labeled OH-RNA; and E shows results of quantitative analysis for the degree of release of OH-RNA supported on DegradaBALL in cells using pearson correlation coefficient.

FIG. 18 is diagrams illustrating results of induction of interferon beta expression of porous silica particles (LEM-S403) carrying RNA according to an embodiment of the present invention.

FIG. 19 is diagrams illustrating results of induction of Viperin expression of porous silica particles (LEM-S403) carrying RNA according to an embodiment of the present invention.

FIG. 20 is diagrams illustrating results of induction of interferon alpha expression of porous silica particles (LEM-S403) carrying RNA according to an embodiment of the present invention.

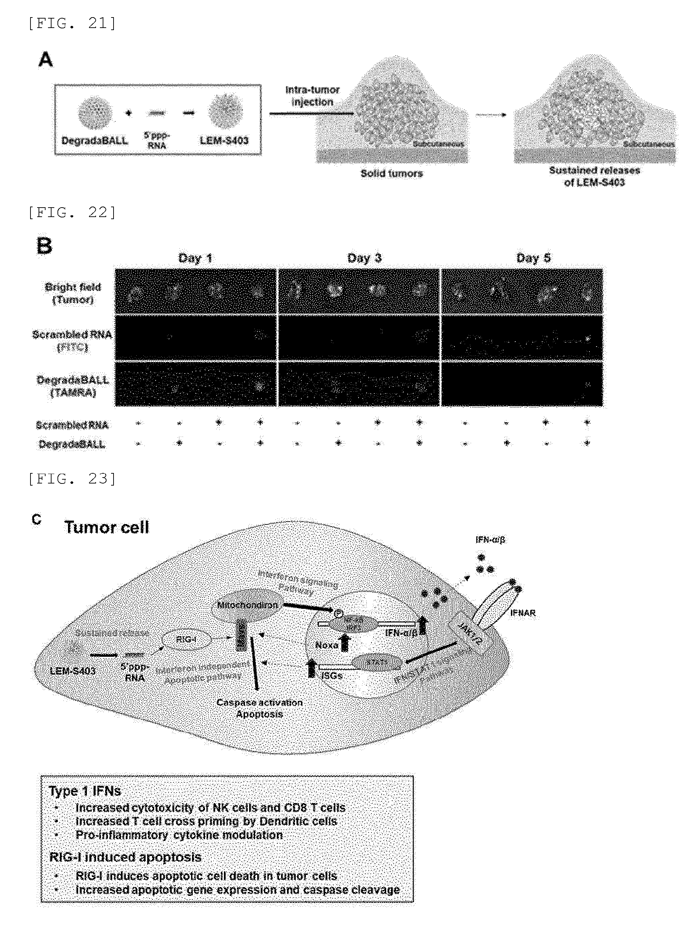

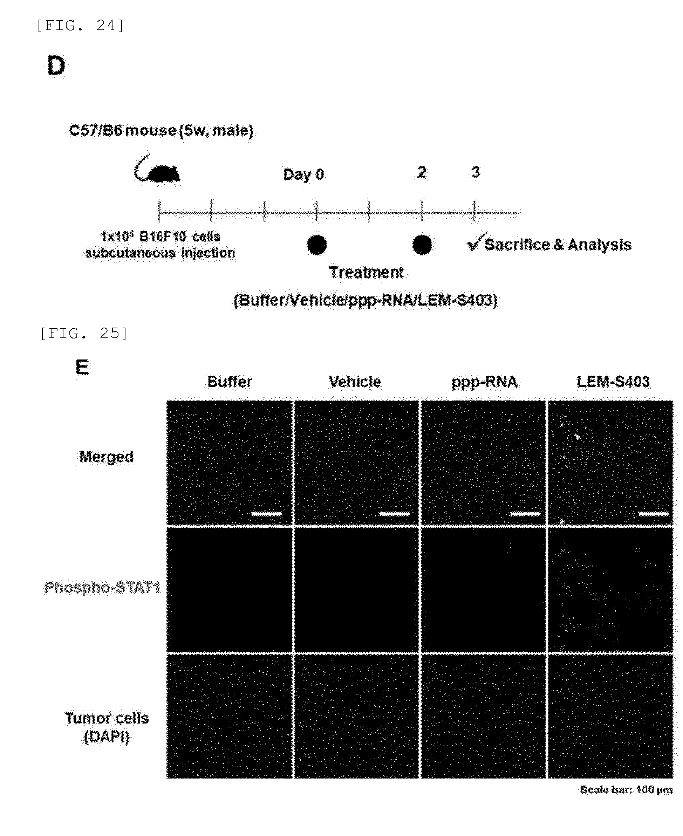

FIGS. 21 to 26 are diagrams illustrating results of induction of interferon-dependent or non-independent tumor cell apoptosis of porous silica particles (LEM-S403) carrying RNA according to an embodiment of the present invention, wherein: A is a schematic diagram representing the delivery of LEM-S403 through intratumoral injection; B shows fluorescence image confirming the tendency of sustained-release of OH-RNA according to the presence or absence of DegradaBALL mixing using FITC-labeled OH-RNA and TAMRA-labeled DegradaBALL, which were analyzed after administration to C57BL/6 mice having planted tumor, followed by isolation of the tumor after 1, 3, and 5 days; C is a schematic diagram illustrating an anti-tumor treatment process through LEM-S403 injection in melanoma tumors, wherein treatment strategies of LEM-S403 are 1) interferon-dependent immune response, and 2) interferon-independent tumor cell death response; D shows a dosing schedule of LEM-S403 mechanism study using C57B6 mice with tumor (B16F10) formed therein, wherein buffer, vehicle (70 pg), ppp-RNA (7 $\mu$g) or LEM-S403 (7 $\mu$g) were administered to mice on 3 days (day 0) and 5 days (day 2) after tumor inoculation (5 mice per group) and, 6 days (day 3) after tumor inoculation, all mice were sacrificed and tumors were isolated, followed by further analysis; E illustrates results of immunofluorescence staining of phosphor-STAT1 in tumors; and F is images of representative tumor tissue fragments through hematoxylin and eosin staining and TUNNEL staining.

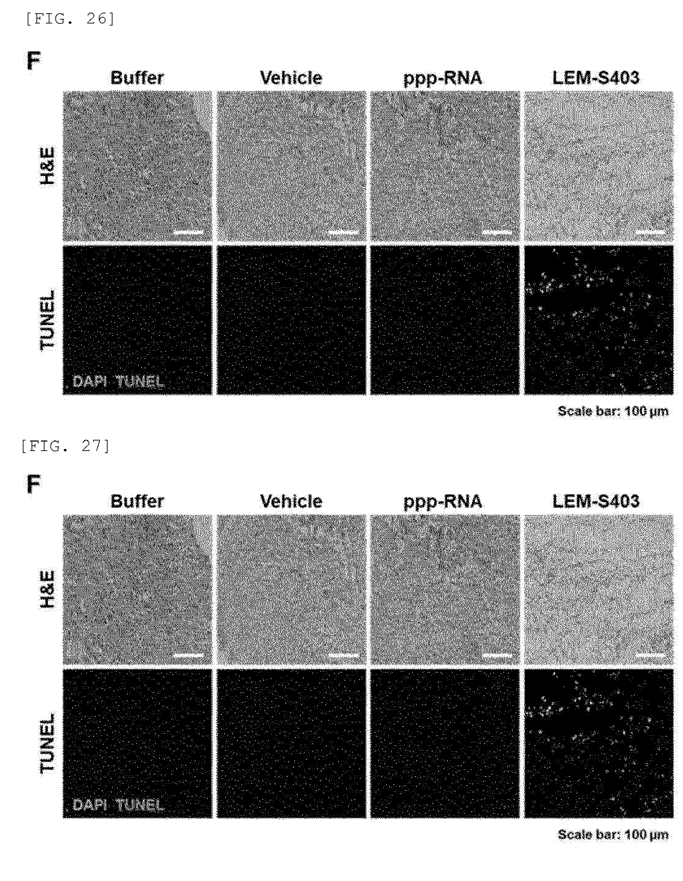

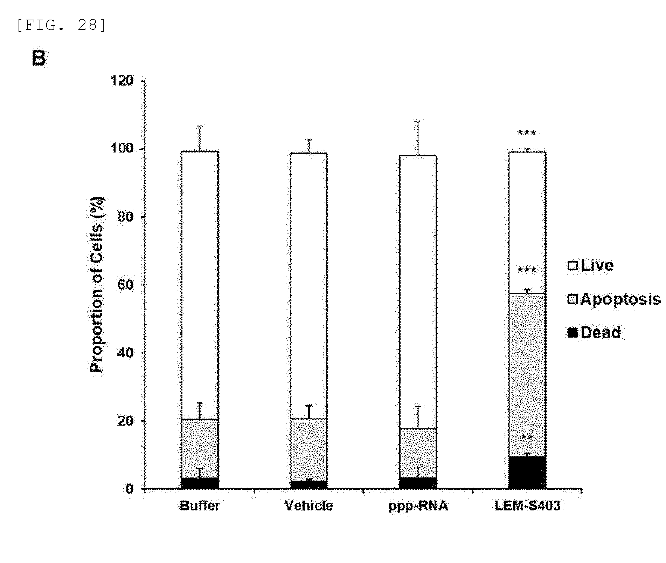

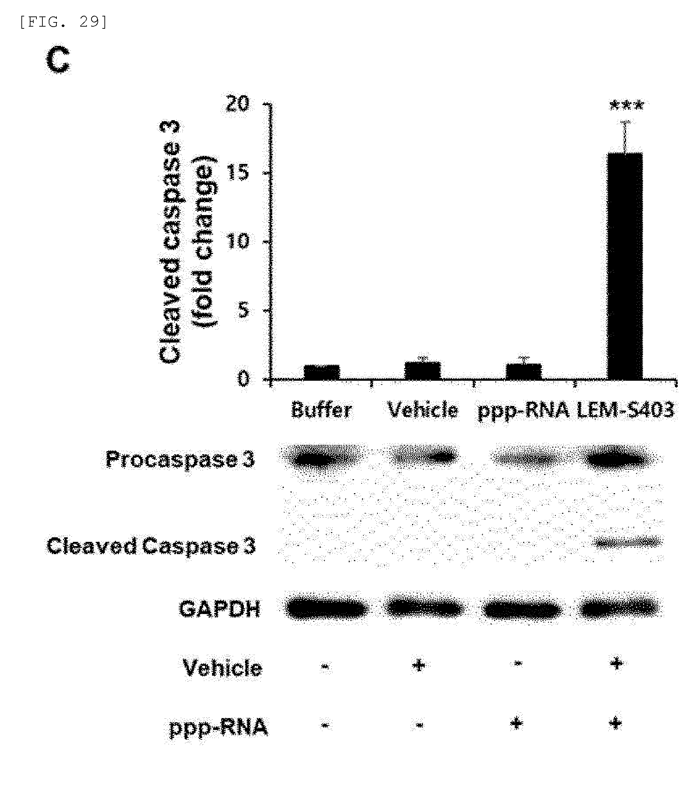

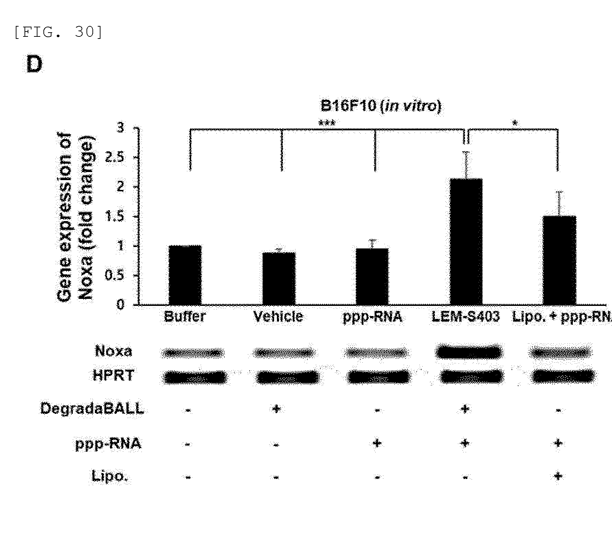

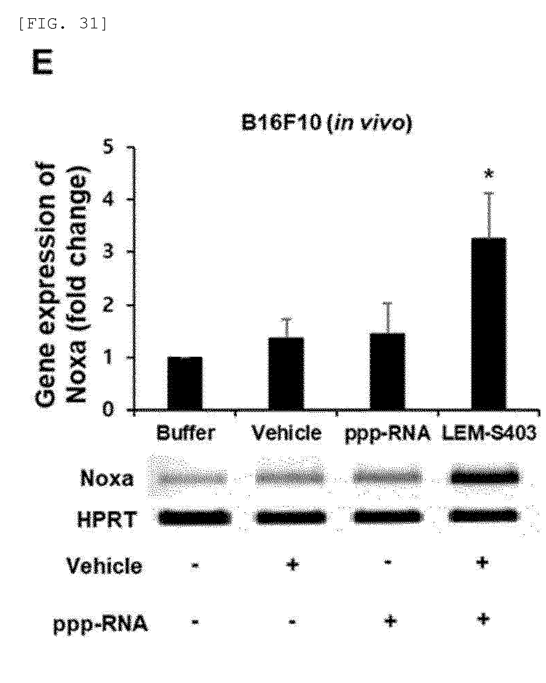

FIGS. 27 to 31 are diagrams illustrating results of induction of interferon-independent tumor cell apoptosis of porous silica particles (LEM-S403) carrying RNA according to an embodiment of the present invention, wherein, after intratumoral injection of buffer, vehicles, ppp-RNA or LEM-S403 into a melanoma mouse model twice (day 0 and day 2), tumor cells isolated on day 3 (day 3) were stained with Annexin V-PI, and wherein: A shows representative images obtained by flow cytometric analysis of tumor cells in each experimental group; B shows ratios of survived cells, cells undergone apoptosis or dead cells of each experimental group; C shows confirmation of capase-3 cleavage in tumors measured by Western-blot; D shows results of RT-PCR analysis of Noxa gene expression after LEM-S403 treatment, using B16F10 cells, wherein mRNA expression was determined 24 hours after treating cells with buffer, ppp-RNA, vehicles, LEM-S403 or Lipofectamine 2000+ppp RNA treatment; and E shows Noxa gene expression in mouse tumors measured by RT-PCR. *$p < 0.05$; **$p < 0.01$; ***$p < 0.001$ vs. other groups (ANOVA).

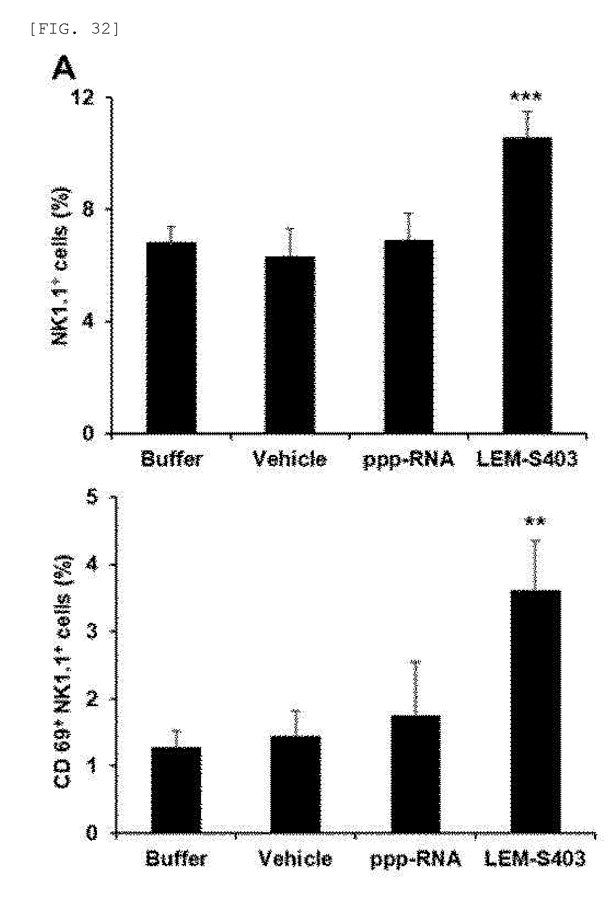

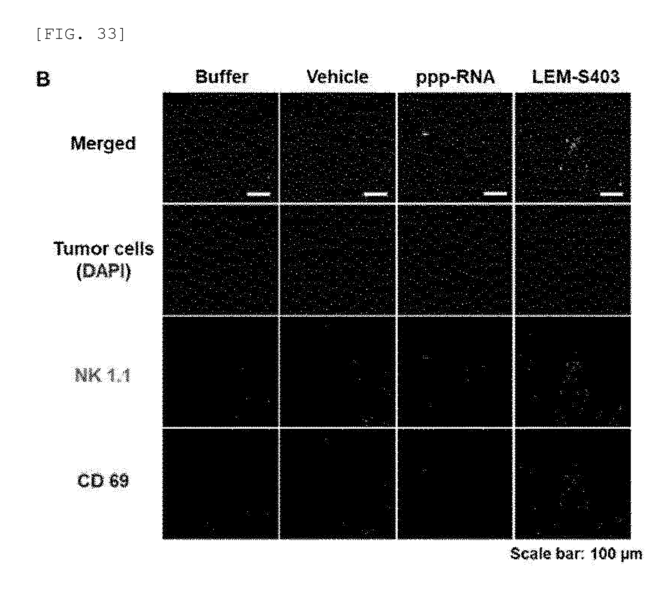

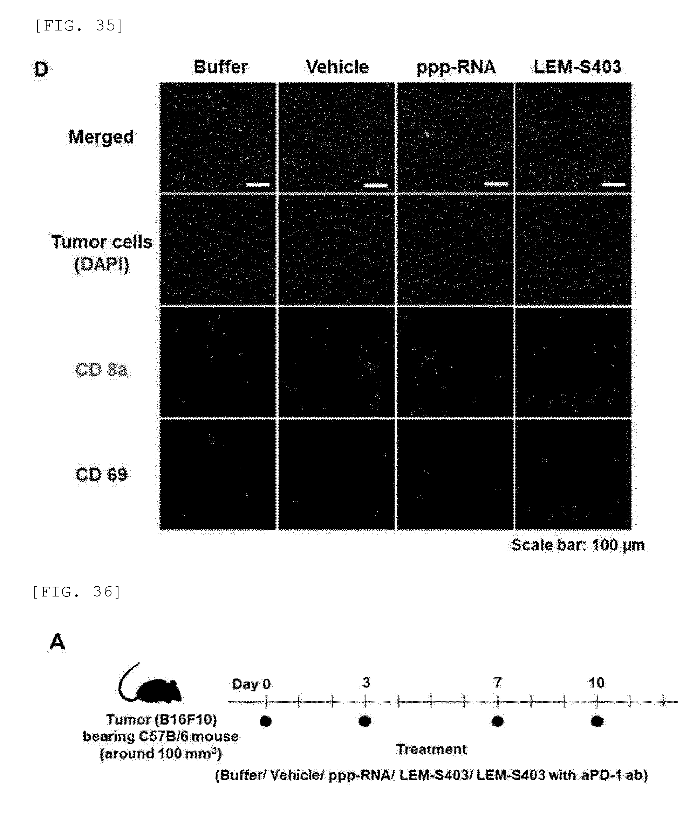

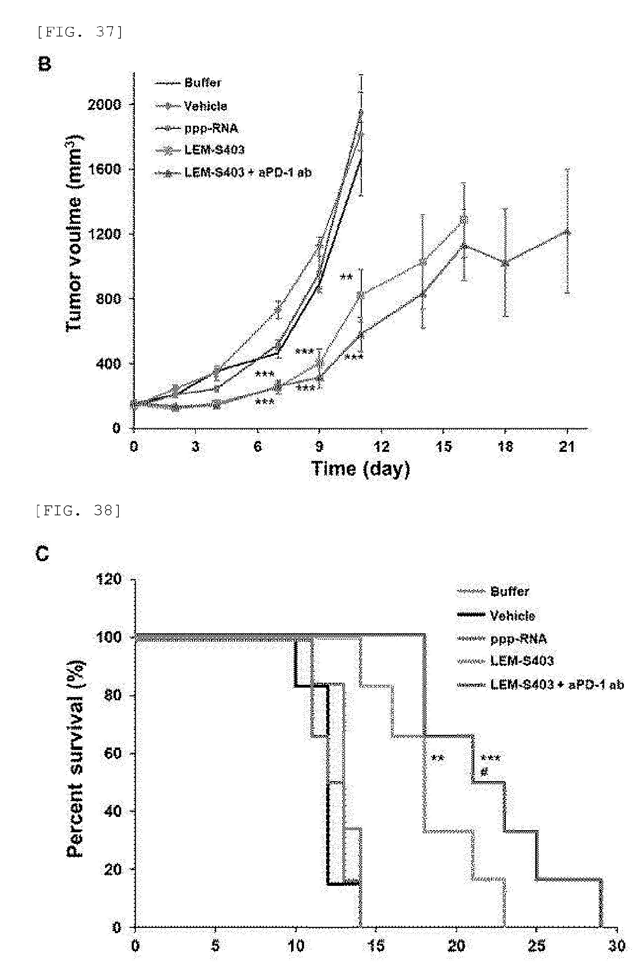

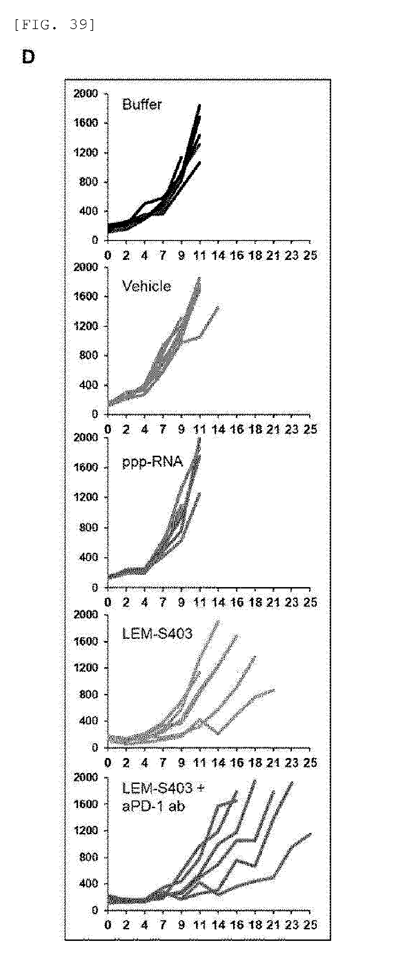

FIGS. 32 to 35 are diagrams illustrating results of evaluation of increase in number and activity of tumor-infiltrating NK cells and CD8+ T cells of the porous silica particles (LEM-S403) carrying RNA according to an embodiment of the present invention, wherein, after inoculating mice with melanoma, buffer, vehicle (70 $\mu$g), ppp-RNA (7 $\mu$g) or LEM-S403 (7 $\mu$g) were administered on 3 days (day 0) and 5 days (day 2) (five (5) mice per group), and wherein, after 6 days (day 3), all mice were sacrificed and tumors were isolated, followed by further analysis, and wherein: A confirmed the distribution of NK cells and CD69-expressing NK cells by flow cytometry of tumor-infiltrating lymphocytes; B shows representative immunofluorescence images of B16F10 tumors indicating NK 1.1 (green) and CD69 (red) expressing cells; C confirmed the distribution of CD8+ T cells and CD69-expressing CD8+ T cells by flow cytometry of tumor-infiltrating lymphocytes; D shows representative immunofluorescence images of B16F10 tumors indicating CD8a (green) and CD69 (red) expressing cells. **p < 0.01; ***p < 0.001 vs. other groups (ANOVA). FIGS. 36 to 39 are diagrams illustrating results of anti-tumor evaluation in the administration of porous silica particles (LEM-S403) carrying RNA alone or in combination with an antibody according to an embodiment of the present invention, wherein: A is a schematic diagram of the experimental design of LEM-S403 alone and in combination with anti-PD-1 in a mouse melanoma (B16F10) model, wherein after inoculation of the tumor in C57B/6 mice, the time point when the tumor volume becomes about 100 mm$^3$ is taken as day 0 and, on 0, 3, 7 and 10 days, buffer (1 × PBS, 50 μL, intratumoral administration), vehicle (70 μg, intratumoral administration), ppp-RNA (7 μg, intratumoral administration), LEM-S403 (7 μg, intratumoral administration) or LEM-S403 (7 μg, intratumoral administration) and anti-PD-1 antibody (10 mg/kg, intraperitoneal administration) were administered; B shows a change in tumor volume measured at 2 and 3 day intervals; C shows changes in survival rates over time for all observed groups (**p < 0.01; ***p < 0.001 vs Buffer, #p < 0.05 vs LEM-S403 by log-rank Mantel-Cox test); and D is a graph showing the tumor volume of each subject in all groups.

FIG. 40 is a schematic diagram of an embodiment in which particles are delivered into the cells.

FIG. 41 is photographs illustrating confirmation of whether or not smooth flow into the cells (B16F10) when the content ratio of the porous silica particles and RNA is differently adjusted according to an embodiment of the present invention.

FIG. 42 is a schematic diagram of porous silica particles according to an embodiment of the present invention.

[Mode for Carrying out Invention]

Hereinafter, the present invention will be described in detail.

The present invention relates to a pharmaceutical composition for improving immune activity.

The pharmaceutical composition for improving immune activity of the present invention may include a blunt-ended hairpin RNA represented by Formula (1) below; and porous silica particles carrying the RNA in pores thereof.

[Formula 1]     5'-P$_a$-((N1-X1)$_b$-N2)-UUCG-(N3-(X2-N4)$_b$)-3'

(wherein P is a phosphoric acid group,

a is an integer of 2 to 5, and b is an integer of 1 to 5,
UUCG is a base to form a loop of the hairpin,
N1 and N2 are 2 to 4 bases selected from G or C, X1 and X2 are 1 to 5 bases selected from A or U, and the plurality of selected bases are the same as or different from one other,
N3 is complementarily bound with N2, X2 is complementarily bound with X1, and N4 is complementarily bound with N1, and
each base repeating b times is the same as or different from one another).
RNA represented by Formula 1 is a blunt ended hairpin structure RNA in which two-stranded polynucleotides are complementarily bound to each other, and the base UUCG in the middle linker portion forms a loop, wherein blunt 5'-triphosphate ends are included to act as a RIG-I agonist.

The composition of the present invention may exhibit immune activity, immune response stimulating or promoting efficacy, which are effects accomplished by stably delivering the supported RNA into the body and releasing the same to a target in order to activate RIG-I signaling pathway, and by promoting the expression of factors such as IFN-α, β, viperin, etc. Therefore, immunity may be activated, thus to exhibit excellent drug efficacy against infectious diseases, cancer, and other immune-related diseases.

Specifically, "immune response" refers to the induction of an antibody and/or immune cell-mediated response specific to an antigen, antigens, allergen(s), drugs or biological agents. Induction of an immune response depends on a number of factors including, for example, an immunogenic configuration of challenged organism, a chemical composition and three-dimensional arrangement of the antigen, allergen, drug or biological agent, and a method and duration of administration of the antigen, allergen, drug or biological agent. The immune response has many aspects, some of which are manifested by cells of the immune system (e.g., B-lymphocytes, T-lymphocytes, macrophages and plasma cells). Cells of the immune system can participate in the immune response through interaction with antigens, allergens or other cells of the immune system, release of cytokines, and responsiveness to these cytokines. The immune response is generally divided into two main categories, that is, humoral and cell-mediated responses. The humoral immune response may involve production of an antibody specific to an antigen, allergen, drug or biological agent. The cell-mediated response may involve delayed hypersensitivity to antigens or allergens and production of cytotoxic effector cells.

Activation or stimulation of the immune system may be mediated by activation of immune effector cells such as lymphocytes, macrophages, dendritic cells, natural killer cells (NK cells) and cytotoxic T lymphocytes (CTL). Further, the immune system activation or stimulation may be mediated by activation and maturation of antigen presenting cells such as dendritic cells. Further, the immune system activation or stimulation may be mediated by blocking an inhibitory pathway such as suppressing immune checkpoint inhibitors.

The infectious disease is a disease caused by infection with a pathogenic antigen, and the pathogenic antigen may include a peptide or protein antigen derived from pathogens associated with infectious diseases, wherein the pathogens may include, for example, a bacterium, a virus, etc., specifically Acinetobacter baumannii, Anaplasma genus, Anaplasma phagocytophilum, Ancylostoma braziliense, Hookworm (Ancylostoma duodenale), hemolytic Akanobacterium haemolyticum, Ascaris lumbricoides, Aspergillus genus, Aspergillus genus, Astroviridae, Babesia genus, Bacillus anthracis, Bacillus cereus, Bartonella henselae, BK virus, Blastocystis hominis, Blastomyces dermatitidis, Borretella pertussis, Borrelia burgdorferi, Borrelia genus, Borrelia spp, Brucella genus, Brugia malayi, Bunyaviridae family, Burkholderia cepacia and other Burkholderia species, Burkholderia mallei, Burkholderia pseudomalei, Caliciviridae family, Campylobacter genus, Candida albicans, Candida spp, Chlamydia trachomatis, Chlamydophila pneumoniae, Chlamydophila psittaci, CJD prion, Clonorchis sinensis, Clostridium botulinum, Clostridium difficile, Clostridium perfringens, Clostridium spp, Clostridium tetani, Coccidioides spp, coronaviruses, Corynebacterium diphtheria, Coxiella burnetii, Crimean-Congo hemorrhagic fever virus, Cryptococcus neoformans, Cryptosporidium genus, Cytomegalovirus (CMV), Dengue viruses (DEN-1, DEN-2, DEN-3 and DEN-4), Dientamoeba fragilis, Ebolavirus (EBOV), Echinococcus genus, Ehrlichia chaffeensis, Ehrlichia ewingii, Ehrlichia genus, Entamoeba histolytica, Enterococcus genus, Enterovirus genus, Enteroviruses, mainly Coxsackie A virus and enterovirus 71 (EV71), Epidermophyton spp, Epstein-Barr Virus (EBV), Escherichia coli 0157:H7, OI11 and O104:H4, epilepsy (Fasciola hepatica) and giant epilepsy (Fasciola gigantica), FFI prion, Filarioidea phase Family (Filarioidea superfamily), Flaviviruses, Tularemia (Francisella tularensis), Fusobacterium genus, Geotrichum candidum, Giardia intestinalis, Evil Gnathostoma spp, GSS prion, Guanarito virus, Haemophilus ducreyi, Haemophilus influenza, Helicobacter pylori, Henipavirus (Hendra virus Nipah virus), hepatitis A virus, hepatitis B virus (HBV), hepatitis C virus (HCV), hepatitis D virus, hepatitis E virus, herpes simplex virus 1 and 2 (HSV-1 and HSV-2), Histoplasma capsulatum, HIV (Human immunodeficiency virus), Hortaea werneckii, Human bocavirus (HBoV), Human herpesvirus 6 (HHV-6) and human herpes virus 7 (HHV-7), human metapneumovirus (hMPV), human papillomavirus (HPV), human parainfluenza virus (HPIV), Japanese encephalitis virus, JC virus, Junin virus, Kingella kingae, Klebsiella granulomatis, Kuru prion, Lassa virus, Legionella pneumophila, Leishmania genus, Leptospira genus, Listeria monocytogenes, Lymphocytic choriomeningitis virus (LCMV), Machupo virus, Malassezia spp, Marburg virus, Measles virus, Metagonimus yokagawai, Microsporidia phylum, Molluscum contagiosum virus (MCV), Mumps virus, Mycobacterium leprae and Mycobacterium lepromatosis, Mycobacterium tuberculosis, Mycobacterium ulcerans, Mycoplasma pneumoniae, Naegleria fowleri, Necator americanus, Neisseria gonorrhoeae, Neisseria meningitides, Nocardia asteroids, Nocardia spp, Onchocerca volvulus, Orientia tsutsugamushi, Orthomyxoviridae family (Influenza), Paracoccidioides brasiliensis, Paracoccidioides spp, Paragonimus westermani, Parvovirus B19, Pasteurella genus, Plasmodium genus, Pneumocystis jirovecii, Poliovirus, Rabies virus, Respiratory syncytial virus (RSV), Rhinovirus, Rickettsia akari, Rickettsia genus, Typhoid rickettsia (Rickettsia prowazekii), Rocky Mountain spotted fever Rickettsia (Rickettsia rickettsia), rash rickettsia virus (Rickettsia typhi) Rift Valley fever virus, Rotavirus, Rubella virus, Sabia virus, Salmonella genus, Sarcoptes scabiei, SARS coronavirus, Schistosoma genus, Shigella genus, Sin Nombre virus, Hantavirus, Sporothrix schenckii, Staphylococcus genus, Streptococcus agalactiae, Streptococcus pneumoniae, Streptococcus pyogenes, Strongyloides stercoralis, Taenia genus, Taenia solium, Tick-borne encephalitis virus (TBEV), Toxocara canis or Toxocara cati, Toxoplasma gondii, Treponema pallidum, Trichinella spiralis, Trichomonas vaginalis, Trichophyton spp, Trichuris trichiura, Trypanosoma brucei, Trypanosoma cruzi, Ureaplasma urealyticum, Varicella zoster virus (VZV), Varicella zoster virus (VZV), small pox major (Variola major) or small pox minor (Variola minor), vCJD prion, Venezuelan equine encephalitis virus, Vibrio cholera, West Nile virus, Western equine encephalitis virus, Wuchereria bancrofti, Yellow fever virus, Yersinia enterocolitica, Yersinia pestis, and Yersinia pseudotuberculosis.

The immune-related diseases may include, for example, sepsis, bedsores, foot ulcers, diabetes, diabetic neuropathy, Alzheimer's disease, Parkinson's disease, dementia, etc., but it is not limited thereto.

The RNA may be, for example, 14 to 100 nt in length. Within the above range, for example, 14 to 100 nt, 14 to 90 nt, 14 to 80 nt, 14 to 70 nt, 14 to 60 nt, 20 to 80 nt, 20 to 60 nt, 20 to 50 nt, 20 to 40 nt, 20 to 35 nt, 25 to 50 nt, 25 to 45 nt, 25 to 35 nt, etc., but it is not limited thereto. When the length is within the above range, excellent immune activity and anticancer activity to be described below may be exhibited, and the particles may exhibit appropriate charge when supported on the particles with appropriate charge.

Specifically, the RNA may be represented by Formula 2 below, and more specifically, may include a sequence selected from the group consisting of SEQ ID NOs: 1 to 25 and 2 to 4 phosphoric acid groups bound to 5'-terminal thereof, and further specifically, may include a sequence selected from the group consisting of SEQ ID NOs: 1 to 25 and 2 to 4 phosphoric acid groups bound to 5'-terminal thereof, and more specifically, may include the sequence of SEQ ID NO: 1 and 3 phosphotric acid groups bound to 5'-terminal thereof.

[Formula 2]   5'-P$_a$-((N1-X1)$_b$-N2)-UUCG-(N3-(X2-N4)$_b$)-3'

(wherein, P is a phosphoric acid group,

a and b are each an integer of 2 to 4,

N1 and N2 are 2 to 4 bases selected from G or C, X1 and X2 are 2 to 4 bases selected from A or U, and the plurality of selected bases are the same as or different from one other,

N3 is complementarily bound with N2, X2 is complementarily bound with X1, and N4 is complementarily bound with N1, and

each base repeating b times is the same as or different from one another).

The porous silica particles may carry the RNA in pores thereof.

The porous silica particles of the present invention are particles based on silica (SiO$_2$) material and have a nano-scale particle size.

The porous silica nanoparticles of the present invention are porous particles, each of which has nano-scale pores, and may carry RNA on an outer surface thereof and/or an inside of the pores.

The porous silica particles may have an average pore diameter of 7 to 25 nm, and the inside of the pores may be positively charged.

The average pore diameter is within the above range, for example, 7 to 25 nm. Within the above range, it may be, for example, 7 to 25 nm, 7 to 23 nm, 10 to 25 nm, 13 to 25 nm, 7 to 20 nm, 7 to 18 nm, 10 to 20 nm, and 10 to 18 nm, but it is not limited thereto.

The RNA has a phosphoric acid group at 5'-terinal thereof, and pppRNA corresponding thereto is usually used while being supported on a carrier such as a liposome, but RNA delivery efficiency is known to be very low. However, the porous silica particles according to the present invention may have an average pore diameter, and the inside of the pores may be charged with a positive charge, whereby the RNA can be sufficiently supported inside the pores and delivered.

The porous silica particles are positively charged inside the pores, and may have a zeta potential of 10 to 70 mV. Within the above range, the zeta potential may be, for example, 10 to 70 mV, 10 to 60 mV, 10 to 50 mV, 10 to 40 mV, 10 to 30 mV, 20 to 70 mV, 20 to 60 mV, 20 to 50 mV, 30 to 60 mV, 15 to 35 mV, etc., but it is not limited thereto.

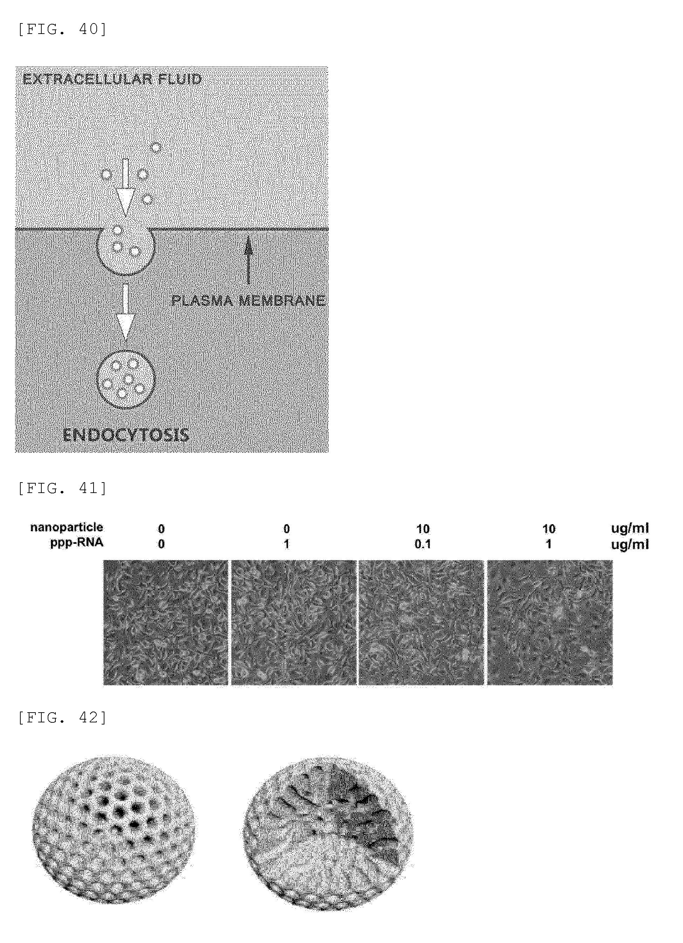

The RNA has a negative charge, and the particles capable of easily carrying RNA should be positively charged. When the charged particles are absorbed into a target cell (e.g., through a process such as Endocytosis illustrated in FIG. 40), the particles entering the cell may have a strong positive charge due to low pH in endosome, which may induce osmosis due to diffusion of water passing a membrane of the endosome, thereby leading to formation of vacuoles. Further, when the particles have a strong positive charge, the cell membrane is wider than when it wraps around the particles, whereby extracellular fluid out of the particles or foreign substances such as various proteins contained therein may be introduced into the target cell together. In such a case, unexpected effects may occur due to inflow of foreign materials or, compared to the case where there is no inflow of foreign materials, the particles may relatively less inflow. Therefore, it may be difficult to generate drug effects by sufficient delivery of the particles. However, the present invention may prevent the problem described above by optimizing the charge of RNA-carrying particles.

Porous silica particles may have a charge of, for example, 5 to 65 mV in a state of carrying RNA. Within the above range, the charge may be, for example, 5 to 65 mV, 5 to 60 mV, 5 to 55 mV, 5 to 50 mV, 5 to 45 mV, 5 to 40 mV, 5 to 35 mV, 5 to 30 mV, 10 to 60 mV, 10 to 55 mV, 10 to 50 mV, 10 to 45 mV, 10 to 40 mV, 10 to 35 mV, 10 to 30 mV, 20 to 50 mV, 20 to 45 mV, 20 to 40 mV, 20 to 35 mV , 20 to 30 mV, 25 to 40 mV, 25 to 35 mV, etc., but it is not limited thereto. In particular, the upper limit of the charge may be 40 mV, specifically 35 mV, more specifically 30 mV, and the lower limit of the charge may be 10 mV, specifically 15 mV, more specifically 20 mV, but it is not limited thereto.

The charge of the particles in a state of carrying RNA may be controlled according to, for example, the charge of the particles not carrying RNA (for example, controlled to have surface modification only), a loading ratio of RNA to the particles, or the like., but it is not limited thereto.

The loading ratio of RNA to the particles may be, for example, a weight ratio of the porous silica particles and RNA of 1: 5 to 20. When a content ratio is within the above range, it is possible to prevent the generation of empty porous silica particles in which RNA is not supported, while sufficiently carrying RNA, whereby the particles having a strong positive charge are prevented from being delivered to the cells. Within the above range, the loading ratio may be, for example, 1: 5 to 20, 1: 5 to 18, 1: 7 to 20, 1: 7 to 18, 1: 7 to 15, 1: 8 to 12, 1: 9 to 11, etc., but it is not limited thereto.

Specifically, in order to administer the composition of the present invention to a subject, the porous silica particles carrying RNA may be dispersed in a dispersion medium, which may be obtained by adding and stirring the porous silica particles and RNA in a dispersion medium. In this regard, if an amount of particles relative to RNA is too large, empty particles not sufficiently carrying RNA may occur, and if the amount of particles relative to RNA is too small, residual RNA not supported by the particles may be generated.

In one embodiment of the present invention, the porous silica particles may have the following specifications in terms of overcoming problems such as formation of vacuoles and inflow of foreign materials during introduction into the cells. For example, a pore size may be 7 to 25 nm, specifically 7 to 20 nm, and more specifically 7 to 15 nm. The particle diameter may be 50 to 500 nm, specifically, 200 to 500 nm, more specifically, 250 to 350 nm. The surface area may be 280 to 680 $m^2/g$, specifically, 280 to 480 $m^2/g$. Zeta potential may be greater than 40 mV, specifically 40 mV to 70 mV before RNA loading. The particles may carry RNA in a weight ratio of 1: 5 to 20, specifically 1: 5 to 15. The zeta potential of the particles after RNA loading may be 40 mV or less, specifically 35 mV or less, more specifically 30 mV or less.

As illustrated in FIG. 42, the porous silica particle may have a plurality of pores, and the pores may extend from the surface to the inside of the particle. Thus, RNA can be sufficiently supported inside the pores.

The porous silica particles of the present invention are biodegradable particles that support RNA and are biodegradable in the body to release RNA when administered into the body. However, the porous silica particles of the present invention are slowly decomposed in the body so that the supported RNA can be released in a sustained manner. For example, t at which an absorbance ratio in Equation 1 below is 1/2 may be 24 or more:

$$[\text{Equation 1}]$$

$$A_t/A_0$$

(wherein $A_0$ is absorbance of the porous silica particles measured by putting 5 ml of suspension containing 1 mg/ml of porous silica particles into a cylindrical permeable membrane having pores with a pore diameter of 50 kDa,

  15 ml of the same solvent as the suspension comes into contact with an outside of the permeable membrane, and
  the inside/outside of the permeable membrane are horizontally stirred at 60 rpm and at 37 °C,
  pH of the suspension is 7.4, and
  $A_t$ indicates absorbance of the porous silica particle measured after lapse of "t" hours since $A_0$ was measured).

The above Equation 1 means what a rate the porous silica particles are degraded in an environment similar to the body.

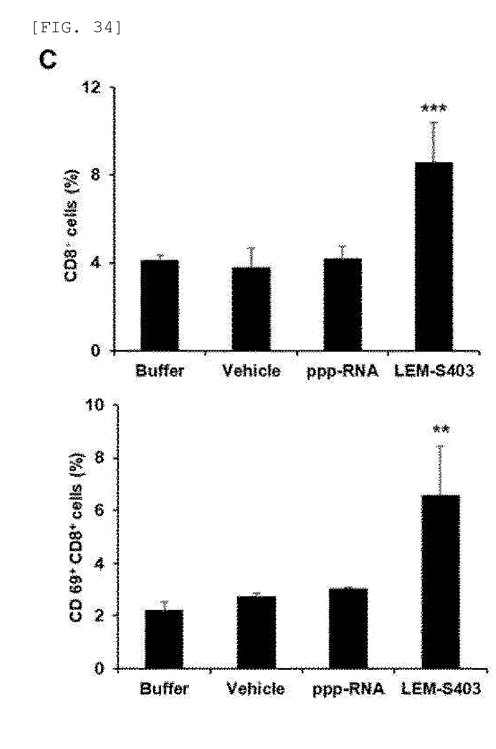

As shown in FIG. 34, for example, absorbance $A_0$ and $A_t$ in the above Equation 1 may be measured after placing porous silica particles and a suspension in a cylindrical permeable membrane and also placing the same suspension outside the permeable membrane.

The porous silica particles of the present invention are biodegradable, and may be slowly degraded in the suspension. The diameter of 50 kDa corresponds to about 5 nm, which allows biodegradable porous silica particles to pass through a permeable membrane having a diameter of 50 kDa, and a cylindrical permeable membrane is under horizontal agitation at 60 rpm to evenly blend the suspension, such that the degraded porous silica particles can come out of the permeable membrane.

The absorbance in the above Equation 1 may be measured, for example, under an environment in which the suspension outside the permeable membrane is replaced with a new suspension. The suspension may be continuously replaced, or replaced every period wherein the period is periodic or irregular. For example, the suspension may be replaced at 1 hour interval, 2 hours interval, 3 hours interval, 6 hours interval, 12 hours interval, 24 hours interval, 2 days interval, 3 days interval, 4 days interval, 7 days interval, etc., within a range of 1 hour to 1 week, but it is not limited thereto.

The absorbance ratio of 1/2 means that the absorbance is half of the initial absorbance after t hours, briefly, that approximately half of the porous silica particles are degraded.

The suspension may be a buffer solution, for example, at least one selected from the group consisting of phosphate buffered saline (PBS) and simulated body fluid (SBF), and more specifically, PBS.

"t" in the above Equation 1 of the present invention, at which the absorbance ratio becomes 1/2, may be 24 or more, for example, t may range from 24 to 120. That is, within the above range, t may range from 24 to 96, 24 to 72, 30 to 70, 40 to 70, 50 to 65, etc., but it is not limited thereto.

With regard to the porous silica particles of the present invention, t at which the absorbance ratio in the above Equation 1 becomes 1/5 may range from 70 to 140. For example, t may range from 80 to 140, 80 to 120, 80 to 110, 70 to 140, 70 to 120, 70 to 110, etc. within the above range, but it is not limited thereto.

With regard to the porous silica particles of the present invention, t at which the absorbance ratio in the above Equation 1 becomes 1/20 may range from 130 to 220. For example, t may range from 130 to 200, 140 to 200, 140 to 180, 150 to 180, etc. within the above range, but it is not limited thereto.

With regard to the porous silica particles of the present invention, t at which the absorbance ratio in the above Equation 1 becomes 0.01 or less may be 250 or more. For example, t may be 300 or more, 350 or more, 400 or more, 500 or more, 1000 or more, etc. and the upper limit may be 2000, but it is not limited thereto.

With regard to the porous silica particles of the present invention, the absorbance ratio and t in the above Equation 1 have high positive correlation. For example, Pearson correlation coefficient may be 0.8 or more, and for example, 0.9 or more and 0.95 or more.

"t" in the above Equation 1 means how fast the porous silica particles are degraded under the environment similar to the body. That is, t may be regulated by adjusting, for example, a surface area, a particle size, a pore diameter, substituents on the surface of the porous silica particles and/or the inside of the pores, compactness of the surface and the like.

For example, the surface area of the particle may be increased to reduce t, or the surface area may be decreased to increase t. The surface area may be regulated by adjusting the particle size and the pore diameter of the particles. Further, if direct exposure of the porous silica particles to the environment (such as solvents) is reduced by placing substituents on the surface of the particles and/or the inside of the pores, t may be increased. Further, when the porous silica particles support or carry RNA, and when increasing affinity between the RNA and the porous silica particles, direct exposure of the porous silica particles to the environment may be reduced, thereby increasing t. In addition, t may be increased by preparing the particles with more compact surface. As described above, various examples of adjusting t in the above Equation 1 have been described, but it is not limited thereto.

The porous silica particles of the present invention may have a spherical shape, but it is not limited thereto.

The porous silica particles of the present invention may have an average diameter of, for example, 50 to 500 nm. For example, the average diameter may range from 50 to 500 nm, 50 to 400 nm, 50 to 300 nm, 100 to 450 nm, 100 to 400 nm, 100 to 350 nm, 100 to 300 nm, 150 to 400 nm. 150 to 350 nm, 200 to 400 nm, 200 to 350 nm, 250 to 400 nm, 180 to 300 nm, 150 to 250 nm, etc. within the above range, but it is not limited thereto.

The porous silica particles of the present invention may have a BET surface area of, for example, 280 to 680 $m^2/g$. For example, the BET surface area may range from 280 $m^2/g$ to 680 $m^2/g$, 280 $m^2/g$ to 600 $m^2/g$, 280 $m^2/g$ to 500 $m^2/g$, 280 $m^2/g$ to 400 $m^2/g$, 300 $m^2/g$ to 650 $m^2/g$, 300 $m^2/g$ to 600 $m^2/g$, 300 $m^2/g$ to 550 $m^2/g$, 300 $m^2/g$ to 500 $m^2/g$, 300 $m^2/g$ to 450 $m^2/g$, 350 $m^2/g$ to 450 $m^2/g$, etc. within the above range, but it is not limited thereto.

Porous silica nanoparticles of the present invention may have a volume per gram, for example, 0.7 to 2.2 ml. For example, the volume may range from 0.7 to 2.0 ml, 0.8 to 2.2 ml, 0.8 to 2.0 ml, 0.9 to 2.0 ml, 1.0 to 2.0 ml, etc. within the above range, but it is not limited thereto. If the volume per gram is too small, a degradation rate may be too high. Further, it is difficult to manufacture excessively large particles or particles having an intact shape.

The porous silica particles may include pores of small pore particles having an average pore diameter of less than 5 nm expanded to an average diameter of 7 to 25 nm. As a result, the pore diameter is large so that large RNA can be carried inside the pore, and the particle diameter itself is not large compared to the pore diameter, so that it is easy to deliver and absorb into the cells.

The porous silica particles of the present invention may be positively charged at an outer surface thereof and/or an inside of the pores. For example, both the surface of the particle and the inside of the pores may be positively charged, or only the surface of the particles or the inside of the pores may be positively charged. The charging may be performed, for example, by the presence of a cationic substituent.

The cationic substance may include, for example, an amino group or any other nitrogen-containing group. Further, the anionic substituent may include, for example, carboxy group (-COOH), sulfonic acid group (-SO$_3$H), thiol group (-SH), etc. as an acidic group, but it is not limited thereto.

Interaction of the porous silica particles with the release environment of the RNA is adjusted to regulate a degradation rate of the nanoparticles, whereby a release rate of the RNA may be regulated. Further, the RNA may be diffused and released from the nanoparticles, wherein adjusting substituents may regulate a binding force of the RNA to the nanoparticles, thereby controlling release of the RNA.

Further, the porous silica particles of the present invention may include substituents for the purposes of: supporting the RNA on the surface of the particles and/or the inside of the pores; delivery of the RNA into a target cell; supporting other substances for other purposes; or binding of additional substituents. Further, the porous silica particles may also include antibodies, ligands, cell permeable peptides, or aptamers bound thereto.

The substituents on the surface of the particles and/or the inside of the pores, charge, binders, etc. described above may be added by, for example, surface modification.

Surface modification may be performed, for example, by reacting a compound having a substituent to be introduced with the particles, wherein the compound may be, for example, alkoxysilane having C1 to C10 alkoxy group, but it is not limited thereto. The alkoxysilane has one or more alkoxy groups, for example, 1 to 3 alkoxy groups. Further, there may be a substituent to be introduced into a site where the alkoxy group is not bound, or a substituent substituted with the same.

The porous silica particles of the present invention may be manufactured, for example, through small pore particle preparation and pore expansion processes and, if necessary, may be manufactured further through calcination, or surface modification process and the like. If both the calcination and the surface modification processes have been implemented, the particles may be surface-modified after calcination.

The small pore particles may be, for example, particles having an average pore diameter of 1 to 5 nm.

The small pore particles may be harvested by adding a surfactant and a silica precursor in a solvent, followed by agitation and homogenization.

The solvent may be water and/or an organic solvent, and the organic solvent may include, for example: ethers such as 1,4-dioxane (particularly cyclic ethers); halogenated hydrocarbons such as chloroform, methylene chloride, carbon tetrachloride, 1,2-dichloroethane, dichloroethylene, trichloroethylene, perchloroethylene, dichloropropane, amyl chloride, 1,2-dibromoethane, etc.; ketones such as acetone, methylisobutylketone, γ-butyrolactone, 1,3-dimethyl-imidazolidinone, methylethylketone, cyclohexanone, cyclopentanone, 4-hydroxy-4-methyl-2-pentanone, etc.; aromatic carbon-based materials such as benzene, toluene, xylene, tetramethylbenzene, etc.; alkyl amides such as N,N-dimethylformamide, N,N-dibutylformamide, N,N-dimethylacetamide, N-methylpyrrolidone, etc.; alcohols such as methanol, ethanol, propanol, butanol, etc.; glycol ethers (cellosolve) such as ethyleneglycol monoethyl ether, ethyleneglycol monomethyl ether, ethyleneglycol monobutyl ether, diethyleneglycol monoethyl ether, diethyleneglycol monomethyl ether, diethyleneglycol monobutyl ether, propyleneglycol monomethyl ether, propyleneglycol monoethyl ether, dipropyleneglycol diethyl ether, triethyleneglycol monoethyl ether, etc.; others such as dimethylacetamide (DMAc), N,N-diethylacetamide, dimethylformamide (DMF), diethylformamide (DEF), N,N-dimethylacetamide (DMAc), N-methylpyrrolidone (NMP), N-ethylpyrrolidone (NEP), 1,3-dimethyl-2-imidazolidinone, N,N-dimethylmethoxyacetamide, dimethyl sulfoxide, pyridine, dimethyl sulfone, hexamethylphosphoamide, tetramethylurea, N-methylcarrolactam, tetrahydrofuran, m-dioxane, P-dioxane, 1,2-dimethoxyethane and the like. Specifically, alcohol, more specifically methanol may be used, but it is not limited thereto.

When using a mixed solvent of water and the organic solvent, a relative ratio of water and organic solvent may be, for example, in a volume ratio of 1: 0.7 to 1.5, for example, 1: 0.8 to 1.3, but it is not limited thereto.

The surfactant may include, for example, cetyltrimethylammonium bromide (CTAB), hexadecyltrimethylammonium bromide (TMABr), hexadecyltrimethylpyridinium chloride (TMPrCl), tetramethylammonium chloride (TMACl), etc., and specifically, CTAB may be used.

The surfactant may be added, for example, in an amount of 1 to 10 g, for example, 1 to 8 g, 2 to 8 g or 3 to 8 g per liter of solvent within the above range, but it is not limited thereto.

The silica precursor may be added after stirring with addition of a surfactant to the solvent. The silica precursor may be, for example, tetramethyl orthosilicate (TMOS), but it is not limited thereto.

The stirring may be conducted, for example, for 10 to 30 minutes, but it is not limited thereto.

The silica precursor may be added in an amount of 0.5 to 5 ml per liter of solvent, for example, 0.5 ml to 4 ml, 0.5 to 3 ml, 0.5 to 2 ml, 1 to 2 ml, etc. within the above range, but it is not limited thereto.

If necessary, sodium hydroxide may further be used as a catalyst, and specifically, may be added under stirring after addition of the surfactant and before addition of the silica precursor to the solvent.

The sodium hydroxide may be added in an amount of 0.5 to 8 ml per liter of solvent, for example, 0.5 to 5 ml, 0.5 to 4 ml, 1 to 4 ml, 1 to 3 ml, 2 to 3 ml, etc. within the above range based on 1 M aqueous sodium hydroxide solution, but it is not limited thereto.

After addition of the silica precursor, the solution may be reacted with stirring. The stirring may be conducted for 2 to 15 hours, for example, 3 to 15 hours, 4 to 15 hours, 4 to 13 hours, 5 to 12 hours, 6 to 12 hours, 6 to 10 hours, etc. within the above range, but it is not limited thereto. If the stirring time (reaction time) is too short, nucleation may be insufficient.

After agitation, the solution may be aged. Aging may be performed for 8 to 24 hours, for example, for 8 to 20 hours, 8 to 18 hours, 8 to 16 hours, 8 to 14 hours, 10 to 16 hours, 10 to 14 hours, etc. within the above range, but it is not limited thereto.

Thereafter, the reaction product may be washed and dried to harvest porous silica particles and, if necessary, separation of unreacted material may proceed before washing.

Separation of the unreacted material may be implemented by separating the supernatant, for example, through centrifugation. For example, centrifugation may be conducted at 6,000 to 10,000 rpm, and the centrifugation time may range from 3 to 60 minutes, for example, 3 to 30 minutes, 5 to 30 minutes, etc. within the above range, but it is not limited thereto.

The washing may be conducted with water and/or an organic solvent. Specifically, since different substances are dissolved in different solvents, water and the organic solvent may be used alternately once or several times, or the washing may be conducted with water or the organic solvent alone once or several times. The several times described above may be 2 times or more and 10 times or less, for example, 3 times or more and 10 times or less, 4 times or more and 8 times or less, 4 times or more and 6 times or less.

The organic solvent may include, for example: ethers such as 1,4-dioxane (particularly cyclic ethers); halogenated hydrocarbons such as chloroform, methylene chloride, carbon tetrachloride, 1,2-dichloroethane, dichloroethylene, trichloroethylene, perchloroethylene, dichloropropane, amyl chloride, 1,2-dibromoethane, etc.; ketones such as acetone, methylisobutylketone, γ-butyrolactone, 1,3-dimethyl-imidazolidinone, methylethylketone, cyclohexanone, cyclopentanone, 4-hydroxy-4-methyl-2-pentanone, etc.; aromatic carbon-based materials such as benzene, toluene, xylene, tetramethylbenzene, etc.; alkyl amides such as N,N-dimethylformamide, N,N-dibutylformamide, N,N-dimethylacetamide, N-methylpyrrolidone, etc.; alcohols such as methanol, ethanol, propanol, butanol, etc.; glycol ethers (cellosolve) such as ethyleneglycol monoethyl ether, ethyleneglycol monomethyl ether, ethyleneglycol monobutyl ether, diethyleneglycol monoethyl ether, diethyleneglycol monomethyl ether, diethyleneglycol monobutyl ether, propyleneglycol monomethyl ether, propyleneglycol monoethyl ether, dipropyleneglycol diethyl ether, triethyleneglycol monoethyl ether, etc.; others such as dimethylacetamide (DMAc), N,N-diethylacetamide, dimethylformamide (DMF), diethylformamide (DEF), N,N-dimethylacetamide (DMAc), N-methylpyrrolidone (NMP), N-ethylpyrrolidone (NEP), 1,3-dimethyl-2-imidazolidinone, N,N-dimethylmethoxyacetamide, dimethyl sulfoxide, pyridine, dimethyl sulfone, hexamethylphosphoamide, tetramethylurea, N-methylcarrolactam, tetrahydrofuran, m-dioxane, P-dioxane, 1,2-dimethoxyethane and the like. Specifically, alcohol, more specifically methanol may be used, but it is not limited thereto.

The washing may be conducted under centrifugation, for example, at 6,000 to 10,000 rpm, and the centrifugation time may range from 3 to 60 minutes, for example, 3 to 30 minutes, 5 to 30 minutes, etc. within the above range, but it is not limited thereto.

Alternatively, the washing may be conducted by filtering out particles through a filter without centrifugation. The filter may have pores in a size of less than or equal to the diameter of the porous silica particles. When filtering the reaction solution with such a filter as described above, only particles remain on the filter, which may be washed by pouring water and/or an organic solvent on the filter.

In the washing, water and the organic solvent may be used alternately once or several times, or the washing may be conducted with water or the organic solvent alone once or several times. The several times described above may be 2 times or more and 10 times or less, for example, 3 times or more and 10 times or less, 4 times or more and 8 times or less, 4 times or more and 6 times or less.

In the particles produced by the above-described method, residual organic substances (surfactants, etc.) used for the reaction may remain on the surface and inside the pores, and washing may be performed to remove the same. Usually, acid treatment (or acidic organic solvent treatment) may be performed to remove such organic substances, but in the present invention, since such acid treatment is not performed, residual organic substances may remain in the pores even after washing.

The drying may be conducted, for example, at 20 to 100 °C, but it is not limited thereto, and may also be conducted in a vacuum state.

Thereafter, the pore of the harvested porous silica particles may be expanded, and such pore expansion may be conducted using a pore swelling agent.

The pore swelling agent may include, for example, trimethylbenzene, triethylbenzene, tripropylbenzene, tributylbenzene, tripentylbenzene, trihexylbenzene, toluene, benzene, etc., and specifically, trimethylbenzene may be used, but it is not limited thereto.

Further, the pore swelling agent used herein may be, for example, N, N-dimethylhexadecylamine (DMHA), but it is not limited thereto.

The pore expansion may be performed, for example, by mixing the porous silica particles in the solvent with a pore swelling agent and heating the mixture to induce reaction.

The solvent may be water and/or an organic solvent, and the organic solvent may include, for example: ethers such as 1,4-dioxane (particularly cyclic ethers); halogenated hydrocarbons such as chloroform, methylene chloride, carbon tetrachloride, 1,2-dichloroethane, dichloroethylene, trichloroethylene, perchloroethylene, dichloropropane, amyl chloride, 1,2-dibromoethane, etc.; ketones such as acetone, methylisobutylketone, cyclohexanone, etc.; aromatic carbon-based materials such as benzene, toluene, xylene, etc.; alkyl amides such as N,N-dimethylformamide, N,N-dibutylformamide, N,N-dimethylacetamide, N-methylpyrrolidone, etc.; alcohols such as methanol, ethanol, propanol, butanol and the like. Specifically, alcohol, more specifically methanol may be used, but it is not limited thereto.

The porous silica particles may be added in a ratio of 10 to 200 g per liter of solvent, for example, 10 to 150 g, 10 to 100 g, 30 to 100 g, 40 to 100 g, 50 to 100 g, 50 to 80 g, 60 to 80 g, etc., within the above range, but it is not limited thereto.

The porous silica particles may be evenly dispersed in a solvent. For example, the porous silica particles may be added to the solvent and ultrasonically dispersed. In the case of using a mixed solvent, the porous silica particles may be dispersed in a first solvent, followed by adding a second solvent thereto.

The pore swelling agent may be added in a ratio of 10 to 200 parts by volume ("vol. parts") based on 100 vol. parts of solvent, for example, 10 to 150 vol. parts, 10 to 100 vol. parts, 10 to 80 vol. parts, 30 to 80 vol. parts, 30 to 70 vol. parts, etc. within the above range, but it is not limited thereto.

The reaction may be carried out at 120 to 190 °C, for example, 120 to 190 °C, 120 to 180 °C, 120 to 170 °C, 130 to 170 °C, 130 to 160 °C, 130 to 150 °C, 130 to 140 °C, etc. within the above range, but it is not limited thereto.

The reaction may be carried out for 6 to 96 hours, for example, 30 to 96 hours, 30 to 80 hours, 30 to 72 hours, 24 to 80 hours, 24 to 72 hours, 36 to 96 hours, 36 to 80 hours, 36 to 72 hours, 36 to 66 hours, 36 to 60 hours, 48 to 96 hours, 48 to 88 hours, 48 to 80 hours, 48 to 72 hours, 6 to 96 hours, 7 to 96 hours, 8 to 80 hours, 9 to 72 hours, 9 to 80 hours, 6 to 72 hours, 9 to 96 hours, 10 to 80 hours, 10 to 72 hours, 12 to 66 hours, 13 to 60 hours, 14 to 96 hours, 15 to 88 hours, 16 to 80 hours, 17 to 72 hours, etc. within the above range, but it is not limited thereto.

The time and temperature may be desirably adjusted within the ranges exemplified above so that the reaction may be carried out sufficiently but not excessively. For example, when the reaction temperature is reduced, the reaction time may be increased, and when the reaction temperature is increased, the reaction time may be shortened. If the reaction is not sufficiently performed, pore expansion may be insufficient. On the other hand, if the reaction proceeds excessively, the particles may collapse due to overexpansion of the pores.

The reaction may be carried out, for example, by gradually raising the temperature. Specifically, the reaction may be carried out by gradually raising the temperature at a rate of 0.5 to 15 °C/min from the room temperature to the above-defined temperature. For example, the temperature may be raised at a rate of 1 to 15 °C/min, 3 to 15 °C/min, 3 to 12 °C/min, 3 to 10 °C/min, etc., but it is not limited thereto.

The reaction may be carried out under stirring. For example, the stirring may be implemented at a speed of 100 rpm or more, and specifically, at a speed of 100 to 1000 rpm, but it is not limited thereto.

After the reaction, the reaction solution may be cooled slowly, for example, by gradually decreasing the temperature. Specifically, the reaction may be carried out by gradually decreasing the temperature at a rate of 0.5 to 20 °C/min from the above-defined temperature to room temperature. For example, the temperature may be decreased at a rate of 1 to 20 °C/min, 3 to 20 °C/min, 3 to 12 °C/min, 3 to 10 °C/min, etc. within the above range, but it is not limited thereto.

If the particles are not washed by acid treatment after the production of the particles, the residual material inside the pores may also participate in the pore expansion, so that the pores may be expanded more sufficiently evenly.

After cooling, the reaction product may be washed and dried to harvest porous silica particles having expanded pores. If necessary, unreacted material may be first separated before washing.

Separation of the unreacted material may be implemented by separating the supernatant, for example, through centrifugation. Herein, centrifugation may be conducted, for example, at 6,000 to 10,000 rpm, and the centrifugation time may range from 3 minutes to 60 minutes. For example, the centrifugation may be conducted for 3 to 30 minutes, 5 to 30 minutes, etc. within the above range, but it is not limited thereto.

The washing may be conducted with water and/or an organic solvent. Specifically, since different substances are dissolved in different solvents, water and the organic solvent may be used alternately once or several times, or the washing may be conducted with water or the organic solvent alone once or several times. The several times described above may be 2 times or more and 10 times or less, for example, 3 times, 4 times, 5 times, 6 times, 7 times, 8 times, etc.

The organic solvent may include, for example: ethers such as 1,4-dioxane (particularly cyclic ethers); halogenated hydrocarbons such as chloroform, methylene chloride, carbon tetrachloride, 1,2-dichloroethane, dichloroethylene, trichloroethylene, perchloroethylene, dichloropropane, amyl chloride, 1,2-dibromoethane, etc.; ketones such as acetone, methylisobutylketone, cyclohexanone, etc.; aromatic carbon-based materials such as benzene, toluene, xylene, etc.; alkyl amides such as N,N-dimethylformamide, N,N-dibutylformamide, N,N-dimethylacetamide, N-methylpyrrolidone, etc.; alcohols such as methanol, ethanol, propanol, butanol and the like. Specifically, alcohol, more specifically methanol may be used, but it is not limited thereto.

The washing may be conducted under centrifugation, for example, at 6,000 to 10,000 rpm, and the centrifugation time may range from 3 to 60 minutes, for example, 3 to 30 minutes, 5 to 30 minutes, etc. within the above range, but it is not limited thereto.

Alternatively, the washing may be conducted by filtering out particles through a filter without centrifugation. The filter may have pores in a size of less than or equal to the diameter of the porous silica particles. When filtering the reaction solution with such a filter as described above, only particles remain on the filter, which may be washed by pouring water and/or an organic solvent on the filter.

In the washing, water and the organic solvent may be used alternately once or several times, or the washing may be conducted with water or the organic solvent alone once or several times. The several times described above may be 2 times or more and 10 times or less, for example, 3 times or more and 10 times or less, 4 times or more and 8 times or less, 4 times or more and 6 times or less.

If the particles are not washed by acid treatment after the production of the particles, residual material inside the pores may remain even after pore expansion, so that the acid treatment may be performed even when washing after pore expansion, but the present invention does not perform acid treatment, instead, residual material can be removed by calcination to be described below.

The drying may be conducted, for example, at 20 to 100 °C, but it is not limited thereto, and may also be conducted in a vacuum state.

Thereafter, the harvested particles may be subjected to calcination, which is a process of heating the particles to remove silanol groups present on the surface of the particles and inside of the pores so as to reduce reactivity of the particles, provide a more compact structure, and remove organic matter filling the pores. For example, the particles may be heated to a temperature of 400 °C or higher. The upper limit of the temperature is not particularly limited but may be 1000 °C, 900 °C, 800 °C, 700 °C, etc. The heating may be conducted, for example, for 3 hours or more or 4 hours or more. The upper limit of the heating time is not particularly limited but may be 24 hours, 12 hours, 10 hours, 8 hours, 6 hours, etc. More particularly, the heating may be conducted at 400 to 700 °C for 3 to 8 hours or at 500 to 600 °C for 4 to 5 hours, but it is not limited thereto.

Removing the organic matter filling the pores can prevent some problems of cytotoxicity or foaming caused by the remaining organic matter.

Then, the harvested porous silica particles may be subjected to surface modification, and the surface modification may be performed on the surface of the particles and/or the inside of the pores. Both the particle surface and the inside of the pores may be surface-modified in the same manner, or may be surface-modified differently.

The particles may be charged through surface modification.

Surface modification may be performed, for example, by reacting a compound having a cationic substituent to be introduced with the particles, wherein the compound may be, for example, alkoxysilane having a C1 to C10 alkoxy group, but it is not limited thereto.

The alkoxysilane has one or more alkoxy groups, for example, 1 to 3 alkoxy groups. Further, there may be a substituent to be introduced into a site where the alkoxy group is not bound, or a substituent substituted with the same.

When alkoxysilane reacts with the porous silicon particles, a covalent bond is formed between a silicon atom and an oxygen atom so that the alkoxysilane may be bound to the surface of the porous silicon particles and/or the inside of the pores. Since the alkoxysilane has a substituent to be introduced, the corresponding substituent may be introduced into the surface of the porous silicon particles and/or the inside of the pores.

The reaction may be carried out by reacting the porous silica particles dispersed in a solvent with alkoxysilane.

The solvent may be water and/or an organic solvent, and the organic solvent may include, for example: ethers such as 1,4-dioxane (particularly cyclic ethers); halogenated hydrocarbons such as chloroform, methylene chloride, carbon tetrachloride, 1,2-dichloroethane, dichloroethylene, trichloroethylene, perchloroethylene, dichloropropane, amyl chloride, 1,2-dibromoethane, etc.; ketones such as acetone, methylisobutylketone, $\gamma$-butyrolactone, 1,3-dimethyl-imidazolidinone, methylethylketone, cyclohexanone, cyclopentanone, 4-hydroxy-4-methyl-2-pentanone, etc.; aromatic carbon-based materials such as benzene, toluene, xylene, tetramethylbenzene, etc.; alkyl amides such as N,N-dimethylformamide, N,N-dibutylformamide, N,N-dimethylacetamide, N-methylpyrrolidone, etc.; alcohols such as methanol, ethanol, propanol, butanol, etc.; glycol ethers (cellosolve) such as ethyleneglycol monoethyl ether, ethyleneglycol monomethyl ether, ethyleneglycol monobutyl ether, diethyleneglycol monoethyl ether, diethyleneglycol monomethyl ether, diethyleneglycol monobutyl ether, propyleneglycol monomethyl ether, propyleneglycol monoethyl ether, dipropyleneglycol diethyl ether, triethyleneglycol monoethyl ether, etc.; others such as dimethylacetamide (DMAc), N,N-diethylacetamide, dimethylformamide (DMF), diethylformamide (DEF), N,N-dimethylacetamide (DMAc), N-methylpyrrolidone (NMP), N-ethylpyrrolidone (NEP), 1,3-dimethyl-2-imidazolidinone, N,N-dimethylmethoxyacetamide, dimethyl sulfoxide, pyridine, dimethyl sulfone, hexamethylphosphoamide, tetramethylurea, N-methylcarrolactam, tetrahydrofuran, m-dioxane, P-dioxane, 1,2-dimethoxyethane and the like. Specifically, alcohol, more specifically methanol may be used, but it is not limited thereto.

The positively charging may be performed by reacting the porous silica particles with alkoxysilane having a basic group such as a nitrogen-containing group, for example, an amino group or an aminoalkyl group. Specifically, N-[3-(trimethoxysilyl)propyl]ethylenediamine, N1-(3-trimethoxysilylpropyl)diethylenetriamine, (3-aminopropyl)trimethoxysilane, N-[3-(trimethoxysilyl)propyl]aniline, trimethoxy[3-(methylamino)propyl]silane, 3-(2-aminoethylamino)propyldimethoxymethylsilane, etc. may be used, but it is not limited thereto.

Further, the surface modification may be performed in combination. For example, surface modification may be performed twice or more on the outer surface of the particles or the inside of the pores. As a specific example, a compound including a carboxyl group may be bound to silica particles having amino groups introduced therein through amide bond in order to change the positively-charged particles to have different surface properties, but it is not limited thereto.

The reaction of the porous silica particles with alkoxysilane may be carried out, for example, under heating. The heating may be conducted at 80 to 180 °C, for example, 80 to 160 °C, 80 to 150 °C, 100 to 160 °C, 100 to 150 °C, 110 to 150 °C, etc. within the above range, but it is not limited thereto.

The reaction of the porous silica particles with alkoxysilane may be carried out for 4 to 20 hours, for example, 4 to 18 hours, 4 to 16 hours, 6 to 18 hours, 6 to 16 hours, 8 to 18 hours, 8 to 16 hours, 8 to 14 hours, 10 to 14 hours, etc. within the above range, but it is not limited thereto.

The reaction temperature, time and an amount of the compound used for surface modification may be desirably selected according to an extent of surface modification. In other words, reaction conditions will vary depending on a level of charge with regard to RNAs. Specifically, by controlling the level of charge of the porous silica particles, a release rate of the RNA may be controlled. For example, if the RNA has a strong negative charge at neutral pH, the reaction temperature may be raised, the reaction time may be extended or the amount of the treated compound may be increased so as to make the porous silica particles to have a strong positive charge, but it is not limited thereto.

Further, the porous silica particles of the present invention may be manufactured through, for example, preparation of small pore particles, pore expansion, surface modification, and internal pore modification.

Preparation of small pore particles and pore expansion may be performed by the above-described processes and, after preparation of the small pore particles and after pore expansion, washing and drying processes may be implemented.

If necessary, unreacted materials may be separated before washing, and separation of the unreacted materials may be conducted by separating the supernatant through centrifugation.

Centrifugation may be conducted at 6,000 to 10,000 rpm, and the centrifugation time may range from 3 to 60 minutes, for example, 3 to 30 minutes, 5 to 30 minutes, etc. within the above range, but it is not limited thereto.

The washing after preparation of small pore particles may be conducted by a method/condition within the above-described range, but it is not limited thereto.

The washing after pore expansion may be conducted under more moderate conditions than the above embodiments. For example, washing may be conducted three times or less, but it is not limited thereto.

The surface modification and internal pore modification may be performed by the above-described processes, respectively. Herein, surface modification and then internal pore modification may be performed in this order, and a washing process may be further conducted between the above two processes.

When the washing is conducted in more moderated conditions after preparation of small pore particles and pore expansion, a reaction solution such as a surfactant used for particle production and pore expansion is filled in the pores so that the inside of the pores is not modified during surface modification and, instead, only the surface of the particles may be modified. Thereafter, the reaction solution inside of the pores may be washed out and removed.

Particle washing between the surface modification and the internal pore modification processes may be carried out using water and/or an organic solvent. Specifically, since different substances are dissolved in different solvents, water and the organic solvent may be used alternately once or several times, or the washing may be conducted with water or the organic solvent alone once or several times. The several times described above may be 2 times or more and 10 times or less, for example, 3 times or more and 10 times or less, 4 times or more and 8 times or less, 4 times or more and 6 times or less.

The washing may be carried out under centrifugation, for example at 6,000 to 10,000 rpm, and the centrifugation time may range from 3 to 60 minutes, for example, 3 to 30 minutes, 5 to 30 minutes, etc. within the above range, but it is not limited thereto.

Alternatively, the washing may be conducted by filtering out particles through a filter without centrifugation. The filter may have pores in a size of less than or equal to the diameter of the porous silica particles. When filtering the reaction solution with such a filter as described above, only particles remain on the filter, which may be washed by pouring water and/or an organic solvent on the filter.

In the washing, water and the organic solvent may be used alternately once or several times, or the washing may be conducted with water or the organic solvent alone once or several times. The several times described above may be 2 times or more and 10 times or less, for example, 3 times or more and 10 times or less, 4 times or more and 8 times or less, 4 times or more and 6 times or less.

The drying may be conducted, for example, at 20 to 100 °C, but it is not limited thereto, and may also be conducted in a vacuum state.

The RNA may be supported on the surface of the porous silica particles and/or inside the pores. Herein, the supporting may be performed, for example, by mixing porous silica particles in a solvent with the RNA.

The solvent may be water and/or an organic solvent, and the solvent may include, for example: ethers such as 1,4-dioxane (particularly cyclic ethers); halogenated hydrocarbons such as chloroform, methylene chloride, carbon tetrachloride, 1,2-dichloroethane, dichloroethylene, trichloroethylene, perchloroethylene, dichloropropane, amyl chloride, 1,2-dibromoethane, etc.; ketones such as acetone, methylisobutylketone, cyclohexanone, etc.; aromatic carbon-based materials such as benzene, toluene, xylene, etc.; alkyl amides such as N,N-dimethylformamide, N,N-dibutylformamide, N,N-dimethylacetamide, N-methylpyrrolidone, etc.; alcohols such as methanol, ethanol, propanol, butanol and the like. Specifically, alcohol, more specifically methanol may be used, but it is not limited thereto.

Further, PBS (phosphate buffered saline solution), SBF (simulated body fluid), borate-buffered saline, tris-buffered saline may be used as the solvent.

The RNA supported on the porous silica particles may be gradually released over an extended time. Such sustained release may be continuous or discontinuous, or linear or nonlinear. Further, the release may vary depending upon characteristics of the porous silica particles and/or interaction between the porous silica particles and the RNA.

The RNA supported on the porous silica particles are released when the porous silica particles are biodegraded. Specifically, the porous silica particles according to the present invention are slowly degraded to allow release of the RNA in a sustained manner. Such release may be controlled by, for example, adjusting surface area, particle size, pore diameter, substituents on the surface of the particles and/or the inside of the pores, surface compactness, etc. with regard to the porous silica particles, but it is not limited thereto.

The RNA supported on the porous silica particles may be released while being separated and diffused from the porous silica particles. Such release is influenced by correlations between the porous silica particles, the RNA and release environment of the same. Therefore, regulating the correlations may control the release of the RNA. For example, by enhancing or weakening a binding force of the porous silica particles to the RNA through surface modification, the release of the RNA may be controlled.

The RNA may be released for a period of, for example, 7 days to 1 year or more, depending on types of treatment to be required, release environments and types of porous silica particles to be used.

Further, if the porous silica particles of the present invention are biodegradable, these can be 100% degraded. Therefore, the RNA supported thereon may be 100% released.

Further, the present invention relates to a pharmaceutical composition for prevention or treatment of cancer.

The pharmaceutical composition of the present invention may include a blunt-ended hairpin RNA represented by Formula 1; and porous silica particles carrying the RNA in pores thereof.

Hairpin RNA and porous silica particles may be within the ranges described above.

The composition of the present invention has anticancer efficacy, which may be attained according to activation of an interferon signaling pathway and/or an interferon-independent cell apoptosis pathway by stably delivering the supported RNA into the body and releasing it to the target (FIG. 23).

Cancer as a target to be prevented or treated using the composition of the present invention may be any cancer that can be prevented or treated through the above pathways, and may include, for example, breast cancer, ovarian cancer, cervical cancer, prostate cancer, testicular cancer, penile cancer, genitourinary tract cancer, testicular tumor, esophageal cancer, laryngeal cancer, gastric cancer, gastrointestinal cancer, skin cancer, keratinocyte cell tumor, follicular carcinoma, melanoma, lung cancer, small cell lung carcinoma, non-small cell lung carcinoma (NSCLC), lung adenocarcinoma, squamous cell carcinoma of the lung, colon cancer, pancreatic cancer, thyroid cancer, papillary cancer, bladder cancer, liver cancer, bile duct cancer, kidney cancer, bone cancer, bone marrow disorder, lymphatic disorder, hair cell cancer, oral and pharyngeal (oral) cancer, lip cancer, tongue cancer, oral cancer, salivary gland cancer, pharyngeal cancer, small intestine cancer, colon cancer, rectal cancer, kidney cancer, prostate cancer, vulvar cancer, thyroid cancer, colorectal cancer, endometrial cancer, uterine cancer, brain cancer, central nervous system cancer, peritoneal cancer, hepatocellular carcinoma, head cancer, neck cancer, Hodgkin disease, leukemia, etc., but it is not limited thereto.

The cancer may be an anticancer drug-resistant cancer, but it is not limited thereto.

The pharmaceutical composition of the present invention may further include a pharmaceutically acceptable carrier, and may be formulated along with such a carrier. As used herein, the term "pharmaceutically acceptable carrier" refers to a carrier or diluent that does not stimulate the organism and does not inhibit biological activities and properties of the administered compound. Pharmaceutical carriers acceptable in the composition formulated as a liquid solution are sterile and biocompatible, and may include saline, sterile water, Ringer's solution, buffered saline, albumin injectable solutions, dextrose solution, maltodextrin solution, glycerol, ethanol, and a mixture of one or more of these components. Further, if necessary, other typical additives such as antioxidants, buffers and bacteriostatic agents may be added. Diluents, dispersants, surfactants, binders and lubricants may also be added to formulate the pharmaceutical composition into injectable formulations, pills, capsules, granules or tablets such as aqueous solutions, suspensions, emulsions and the like.

The pharmaceutical composition of the present invention is applicable in a form of any formulation containing the nucleic acid molecule of the present invention as an active ingredient, and may be prepared in oral or parenteral formulations. The pharmaceutical formulations of the present invention may include forms suitable for oral, rectal, nasal, topical (including the cheek and sublingual), subcutaneous, vaginal or parenteral (intramuscular, subcutaneous) administration. Alternatively, forms suitable for administration by inhalation or insufflations may also be included.

The pharmaceutical composition of the present invention is administered in a pharmaceutically effective amount. Effective dose levels may be determined depending on types of disease of the patient, severity, activity of drug, sensitivity to drug, administration time, administration route and rate of release, duration of treatment, factors including concurrent medications, and other factors well known in the medical field. The pharmaceutical composition of the present invention may be administered as an individual therapeutic agent or in combination with other therapeutic agents, may be administered sequentially or simultaneously with conventional therapeutic agents, and may be administered in single or multiple doses. Taking all of the above factors into consideration, it is important to administer the pharmaceutical composition in an amount that can achieve maximum effects with a minimum amount without side effects, which may be easily determined by those skilled in the art.

The dosage of the pharmaceutical composition according to the present invention may vary widely depending on the weight, age, sex, health conditions or diet of a patient, administration time, administration method, excretion rate and severity of the disease, and the appropriate dosage depends on, for example, an amount of drug accumulated in the patient's body and/or specific efficacy of the carrier of the present invention used. For example, the amount may be from 0.01 $\mu$g to 1 g per kg of body weight. Further, the pharmaceutical composition of the present invention may be administered once or several times per unit time during unit periods of time such as daily, weekly, monthly or yearly, or may be continuously administered using an infusion pump for a long time. The number of repeated administration doses is determined in consideration of a residential time of drug in the body, a drug concentration in the body, etc. Even after treatment according to the course of disease treatment, the composition may be further administered for preventing recurrence, i.e., relapse of the disease.

The pharmaceutical composition of the present invention may further include a compound to maintain/increase one or more of active ingredients exhibiting the same or similar functions in relation to treatment of wounds or the solubility and/or absorption of at least one active ingredient.

Further, the pharmaceutical composition of the present invention may be formulated using any method known in the art to allow rapid, sustained or delayed release of the active ingredient after administration to a mammal. The formulation may be produced in a form of powders, granules, tablets, emulsions, syrups, aerosols, soft or hard gelatin capsules, sterile injectable solutions, sterile powders.

Hereinafter, the present invention will be described in detail with reference to the following examples.

**Example**

**Example 1. Porous silica particles (DDV or DegradaBALL)**

**1. Preparation of porous silica particles**

**(1) Preparation of porous silica particles**

**1) Preparation of small pore particles**

960 mL of distilled water (DW) and 810 mL of MeOH were put into a 2 L round bottom flask. 7.88 g of CTAB was added to the flask, followed by rapid addition of 4.52 mL of 1 M NaOH under stirring. After adding a homogeneous mixture while stirring for 10 minutes, 2.6 mL of TMOS was further added. After stirring for 6 hours to mix uniformly, the reaction solution was aged for 24 hours.

Then, the reaction solution was centrifuged at 8000 rpm and 25 °C for 10 minutes to remove the supernatant, centrifuged at 8000 rpm and 25 °C for 10 minutes, and washed five times with ethanol and distilled water alternately.

Thereafter, the resultant product was dried in an oven at 70 °C to harvest 1.5 g of powdery small pore porous silica particles (pore average diameter of 2 nm and particle size of 200 nm).

**2) Pore expansion**

1.5 g of small pore porous silica particle powder was added to 10 ml of ethanol and subjected to ultrasonic dispersion, and 10 ml of water and 10 ml of TMB (trimethyl benzene) were further added, followed by ultrasonic dispersion.

Thereafter, the dispersion was placed in an autoclave and reacted at 160 °C for 48 hours.

The reaction was initiated at 25 °C and performed while raising the temperature at a rate of 10 °C/min, then slowly cooled in an autoclave at a rate of 1 to 10 °C/min.

The cooled reaction solution was centrifuged at 8000 rpm for 10 minutes at 25 °C to remove the supernatant, and centrifuged at 8000 rpm for 10 minutes at 25 °C and washed five times with ethanol and distilled water alternately.

Then, the product was dried in an oven at 70 °C to harvest powdery porous silica particles (pore diameter of 10 to 15 nm, and particle size of 200 nm).

**3) Calcination**

The porous silica particles prepared in 2) were put in a glass vial, heated at 550 °C for 5 hours, and cooled slowly to room temperature after completing the reaction to prepare particles.

**(2) Preparation of porous silica particles**

Porous silica particles were prepared by the same method as Example 1-1-(1), except that the reaction conditions at the time of pore expansion were changed to 140 °C and 72 hours.

**(3) Preparation of porous silica particles (10 L scale)**

Porous silica particles were prepared by the same method as Example 1-1-(1), except that a 5 times larger container was used and each material was used in a 5 times capacity.

**(4) Preparation of porous silica particles (particle size of 300 nm)**

Porous silica particles were prepared by the same method as Example 1-1-(1), except that 920 ml of distilled water and 850 ml of methanol were used to prepare the small pore particles.

**(5) Preparation of porous silica particles (particle size of 500 nm)**

Porous silica particles were prepared by the same method as Example 1-1-(1), except that 800 ml of distilled water, 1010 ml of methanol, and 10.6 g of CTAB were used to prepare the small pore particles.

**(6) Preparation of porous silica particles (particle size of 1000 nm)**

Porous silica particles were prepared by the same method as Example 1-1-(1), except that 620 ml of distilled water, 1380 ml of methanol, and 7.88 g of CTAB were used to prepare the small pore particles.

**(7) Preparation of porous silica particles (pore diameter of 4 nm)**

Porous silica particles were prepared by the same method as Example 1-1-(1), except that 2.5 mL of TMB was used for pore expansion.

**(8) Preparation of porous silica particles (pore diameter of 7 nm)**

Porous silica particles were prepared by the same method as Example 1-1-(1), except that 4.5 mL of TMB was used for pore expansion.

**(9) Preparation of porous silica particles (pore diameter of 17 nm)**

Porous silica particles were prepared by the same method as Example 1-1-(1), except that 11 mL of TMB was used for pore expansion.

**(10) Preparation of porous silica particles (pore diameter of 23 nm)**

Porous silica particles were prepared by the same method as Example 1-1-(1), except that 12.5 mL of TMB was used for pore expansion.

**(11) Preparation of porous silica particles**

Porous silica particles were prepared by the same method as Example 1-1-(1), except that 900 ml of distilled water, 850 mL of methanol and 8 g of CTAB were used in preparation of small pore particles.

**2. Surface modification of porous silica particles**

    (1) The porous silica particles of Example 1-1-(1) were reacted with (3-aminopropyl)triethoxysilane (APTES) to be positively charged.

Specifically, 100 mg of porous silica particles were dispersed in a 10 mL toluene in a 100 mL round bottom flask with a bath sonicator. Then, 1 mL of APTES was added and stirred at 400 rpm and 130 °C for 12 hours.

After the reaction, the product was slowly cooled to room temperature and centrifuged at 8000 rpm for 10 minutes to remove the supernatant, further centrifuged at 8000 rpm and 25 °C for 10 minutes, and then washed five times with ethanol and distilled water alternately.

Thereafter, the product was dried in an oven at 70 °C to harvest powdery porous silica particles having an amino group on the surface thereof and inside of the pores.

(2) The product of Example 1-1-(11) was subjected to surface modification by the same method described above except of using 1.8 mL of APTES, thereby obtaining powdery porous silica particles having amino groups on surface of the particle and inside the pore.

### 3. Identification of particle formation and pore expansion

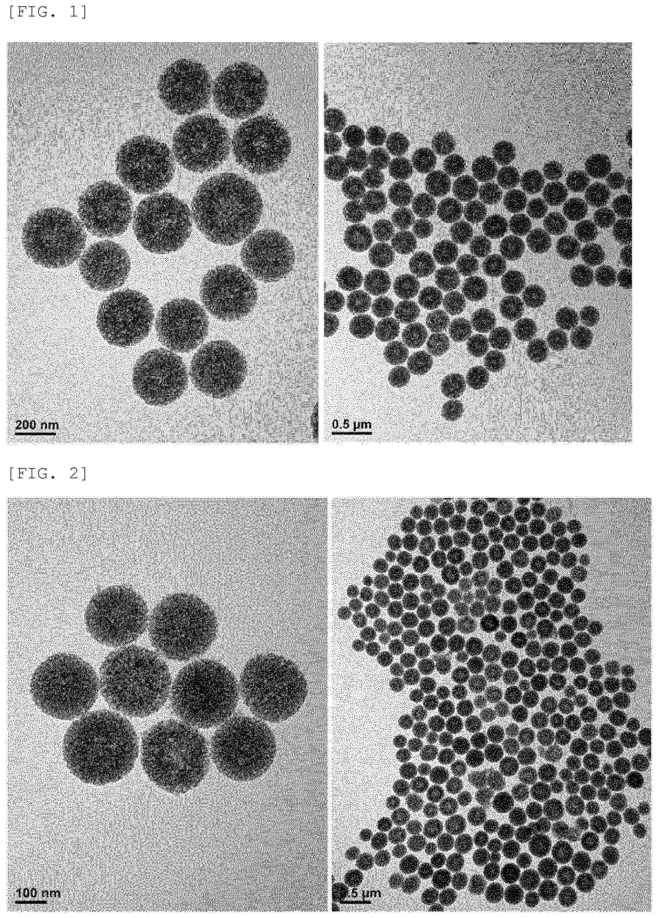

Small pore particles and porous silica particles prepared in Experimental Examples 1-1-(1) to (3) were observed under a microscope to determine whether the small pore particles were uniformly formed or the pores were sufficiently expanded to uniformly form the porous silica particles (FIGS. 1 to 4).

FIG. 1 is photographs of the porous silica particles in Example 1-1-(1), and FIG. 2 is photographs of the porous silica particles in Example 1-1-(2), and from these drawings, it can be seen that spherical porous silica particles having sufficiently expanded pores were formed evenly.

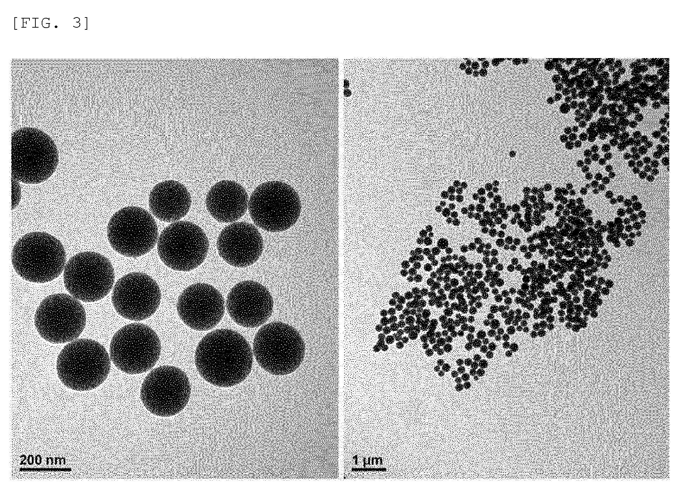

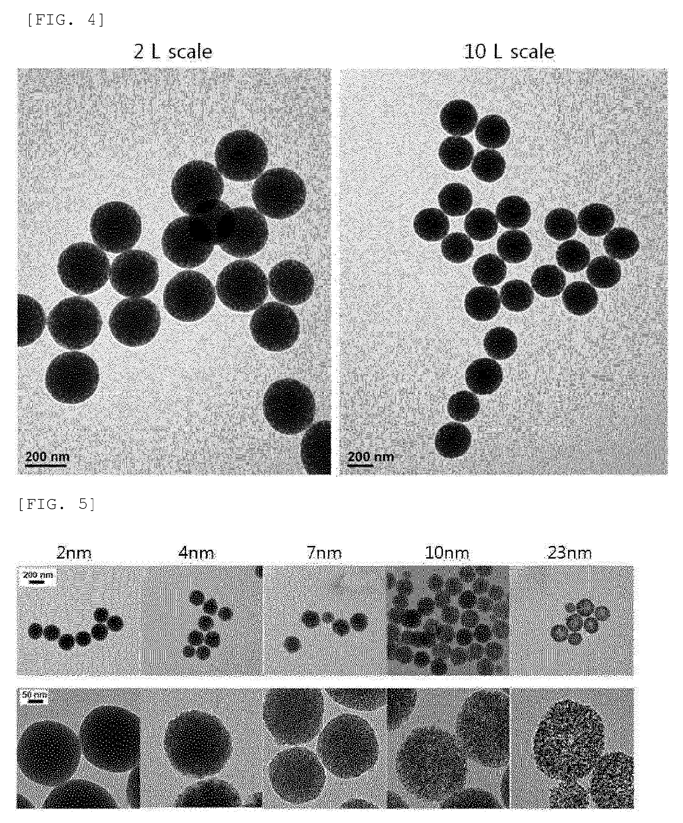

FIG. 3 is photographs of the small pore particles in Example 1-1-(1), and FIG. 4 is comparative photographs of the small pore particles in Examples 1-1-(1) and 1-1-(3), and from these drawings, it can be seen that spherical small pore particles were formed evenly.

### 4. Calculation of BET surface area and pore diameter

Surface areas and pore diameter of the small pore particles in Example 1-1-(1) and the porous silica particles of Examples 1-1-(1), (7), (8), (10) and (11) were calculated. The surface areas were calculated by Brunauer-Emmett-Teller (BET) method, and the pore diameter distributions were calculated by Barrett-Joyner-Halenda (BJH) method.

Micrographs of the particles are shown in FIG. 5, and the calculation results are shown in Table 1 below.

[TABLE 1]

| Section | Pore diameter (nm) | BET surface area (m$^2$/g) |
|---|---|---|
| Small pore particle in Example 1-1-(1) | 2.1 | 1337 |
| Example 1-1-(7) | 4.3 | 630 |
| Example 1-1-(8) | 6.9 | 521 |
| Example 1-1-(1) | 10.4 | 486 |
| Example 1-1-(10) | 23 | 395 |
| Example 1-1-(11) | 12.3 | 379 |

### 5. Identification of biodegradability

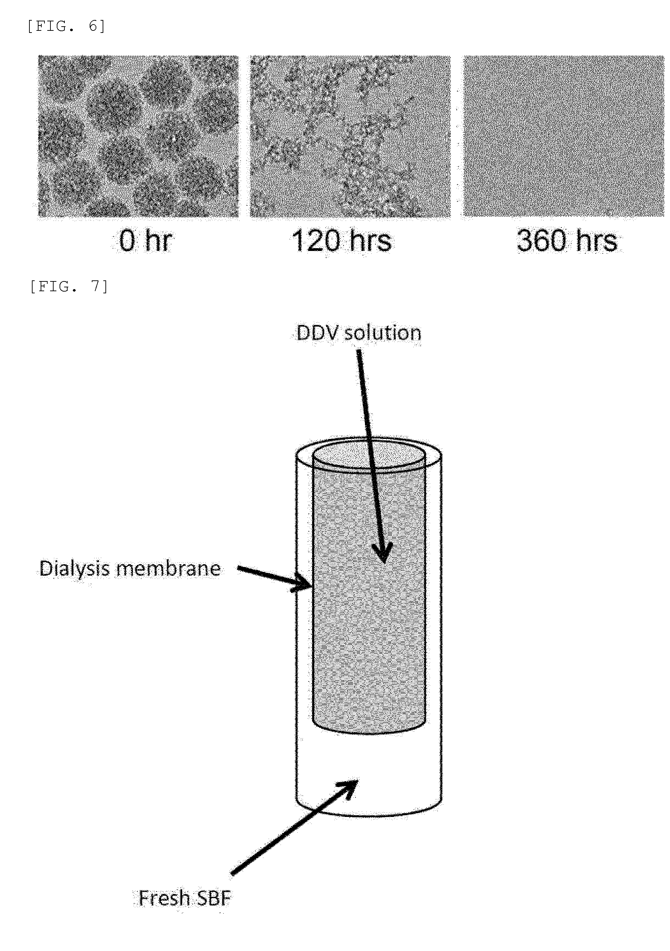

In order to identify biodegradability of the porous silica particles in Example 1-1-(1), biodegradability at 37 °C in SBF (pH 7.4) was observed under a microscope at 0 hours, 120 hours and 360 hours, and results thereof are shown in FIG. 6.

Referring to FIG. 6, it can be seen that the porous silica particles are biodegraded and almost degraded after 360 hours.

### 6. Measurement of absorbance ratio

Absorbance ratio over time was measured according to Equation 1 below.

$$[\text{Equation 1}]$$

$$A_t/A_0$$

(wherein $A_0$ is absorbance of the porous silica particles measured by putting 5 ml of suspension containing 1 mg/ml of the porous silica particles into a cylindrical permeable membrane having pores with a pore diameter of 50 kDa,

15 ml of the same solvent as the suspension comes into contact with an outside of the permeable membrane, and the inside/outside of the permeable membrane are horizontally stirred at 60 rpm and 37 °C, and

$A_t$ indicates absorbance of the porous silica particles measured after lapse of "t" hours since $A_0$ was measured).

Specifically, 5 mg of porous silica particle powder was dissolved in 5 ml of SBF (pH 7.4). Thereafter, 5 ml of porous silica particle solution was placed in a permeable membrane having pores with a pore diameter of 50 kDa shown in FIG. 7. 15 ml of SBF was added to the outer membrane, and the SBF on the outer membrane was replaced every 12 hours. Degradation of the porous silica particles was performed at 37 °C under horizontal stirring at 60 rpm.

Then, the absorbance was measured by UV-vis spectroscopy and analyzed at $\lambda$ = 640 nm.

**(1) Measurement of absorbance ratio**

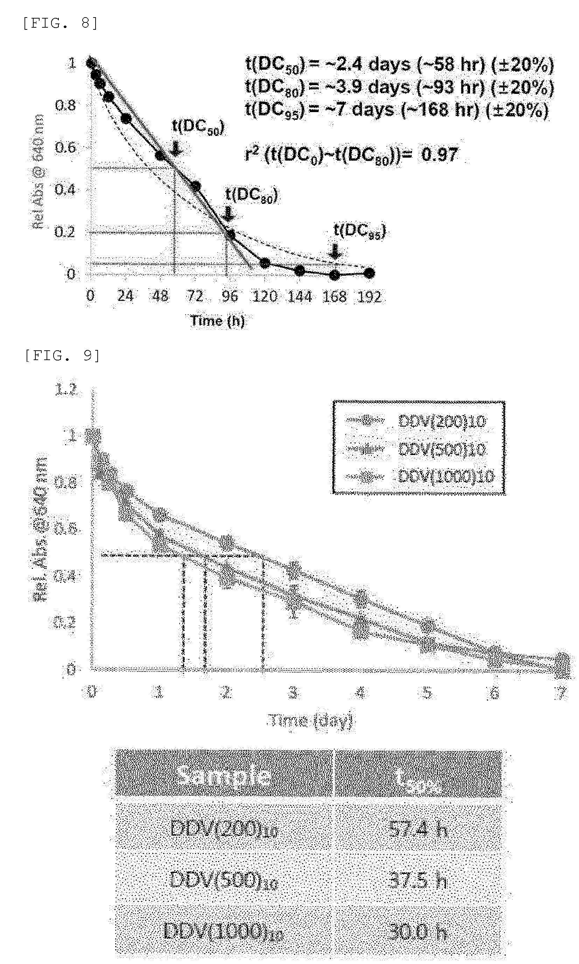

Absorbance ratio of the porous silica particles in Example 1-1-(1) was measured according to the above method, and results thereof are shown in FIG. 8.

Referring to FIG. 8, it can be seen that t, at which the absorbance ratio becomes 1/2, is about 58 hours to demonstrate very slow degradation.

**(2) Particle size**

Absorbances of the porous silica particles in Examples 1-1-(1), (5) and (6) were measured according to Equation 1 above, and results thereof are shown in FIG. 9 (SBF used as the suspension and the solvent).

Referring to FIG. 9, it can be seen that t is decreased as the particle size is increased.

**(3) Average pore diameter**

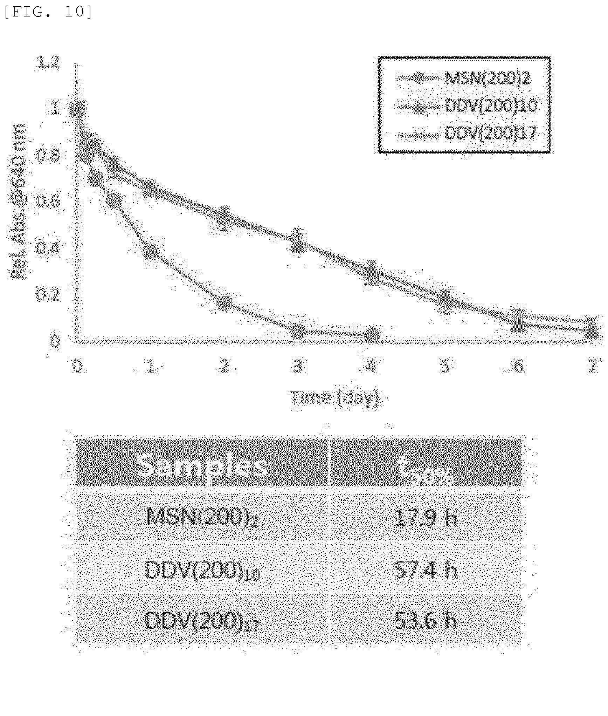

Absorbances of the porous silica particles in Examples 1-1-(1) and (9) and the small pore porous silica particles in Example 1-1-(1) as a control were measured according to Equation 1 above, and results thereof are shown in FIG. 10 (SBF used as the suspension and the solvent).

Referring to FIG 10, it can be seen that the porous silica particles of the inventive example have a significantly larger t than the control.

**(4) pH**

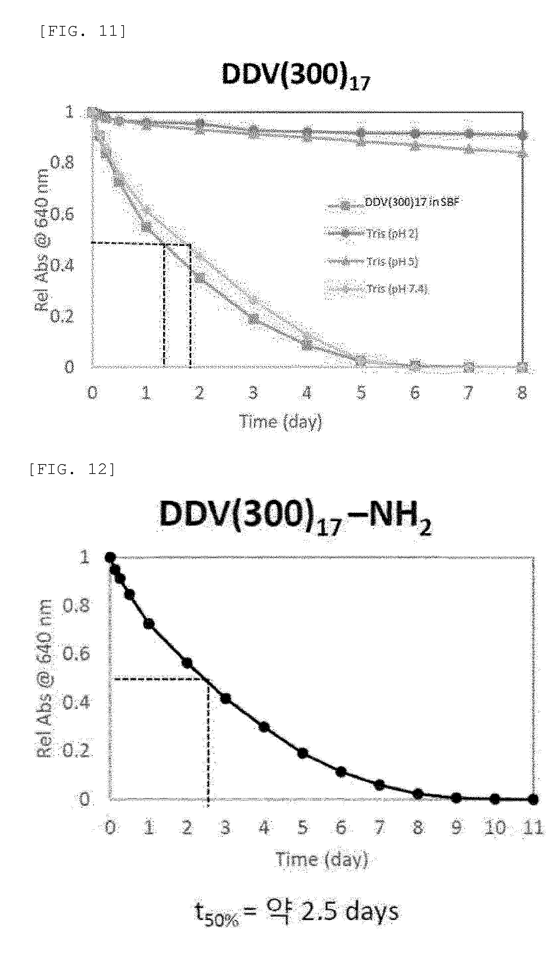

Absorbance of the porous silica particles in Example 1-1-(4) for each pH was measured. The absorbance was measured in SBF and in Tris at pH 2, 5, and 7.4, and results thereof are shown in FIG. 11.

Referring to FIG 11, it could be seen that, although there is a difference in t in relation to pH, t at which all absorbance ratio becomes 1/2 was 24 or more.

**(5) Charging**

Absorbance of the porous silica particles in Example 1-2-(1)-1 was measured, and results thereof are shown in FIG. 12 (Tris (pH 7.4) used as the suspension and the solvent).

Referring to FIG 12, it could be seen that t at which the absorbance ratio of the positively charged particles becomes 1/2 was 24 or more.

**7. RNA loading and release**

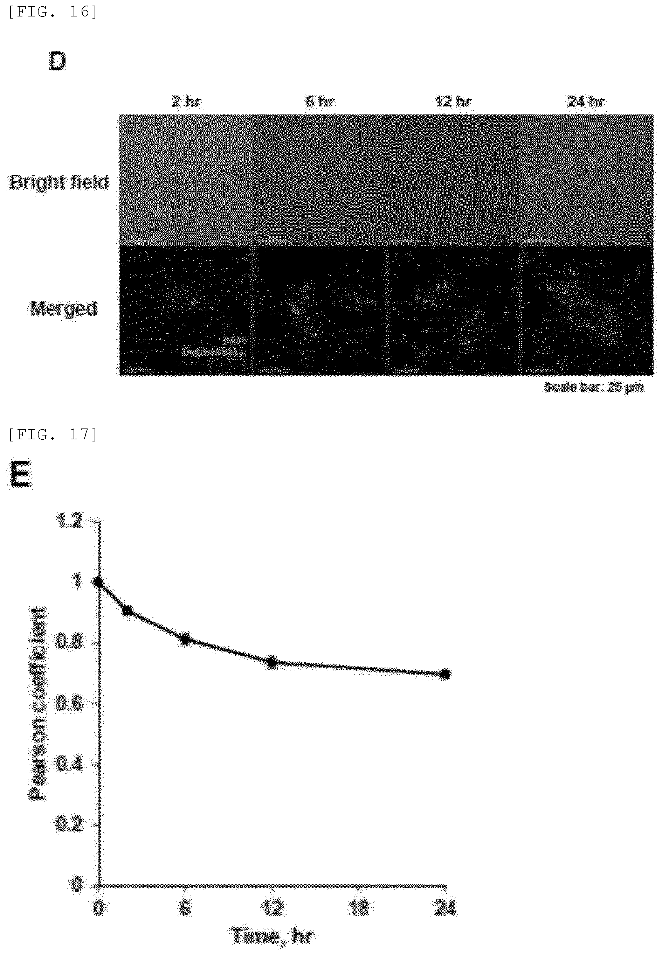

Synthetic RIG-I ligand (5'-triphosphate hairpin RNA, ppp-RNA, SEQ ID NO: 1) and control hairpin RNA (OH-RNA) were synthesized (FIG. 14B). 40, 30, 20, 10, 5 $\mu$g of the porous silica particles (DegradaBALL) of Example 1-1-(11), respectively, were mixed in 1 $\mu$g of ppp-RNA and left at room temperature for 10 minutes, followed by centrifugation and taking the supernatant. As a result of detecting ppp-RNA in the supernatant by SDS-PAGE, it was confirmed that no ppp-RNA was detected until a weight ratio is 1: 10 (ppp-RNA:DegradaBALL) (FIG. 15C). Based on this result, a loading capacity of DegradaBALL was set to 10% in subsequent experiments. After loading OH-RNA labeled with Cy5 on DegradaBALL labeled with FITC, A549 cells were treated with an OH-RNA at a concentration of 5 nM for 2 hours. After replacing the medium with a new medium, culturing was continued till a predetermined time point (6, 12, and 24 hours after treatment), followed by observation through a high-resolution microscope. Thereafter, the culture was subjected to quantitative analysis (FIGS. 16, 17D and E). From this, it was confirmed that the OH-RNA supported on the DegradaBALL moved into the cells, and then slowly released from the DegradaBALL over 24 hours.

**8. Immune activation effect**

25000 cells (A549 cells, medium RPMI1640, 10% FBS, 1% P/S) per well of a 24-well plate were dispensed and incubated overnight in a $CO_2$ 5%, 37°C incubator. The following day, as shown in the table, each sample was mixed under preset conditions and left for 30 minutes to prepare a mixture for gene introduction. Gene introduction was prepared by mixing the prepared mixture in a serum-free culture solution (RPMI1640, 1% P/S).

[TABLE 2]

|  | Control | Group 1 | Group 2 | Group 3 | Group 4 | Group 5 |
|---|---|---|---|---|---|---|
| ppp-RNA |  |  |  | 850 ng | 850 ng | 850 ng |
| DegradaBALL |  |  | 15 μg |  |  | 15 μg |
| LNP |  | 0.5 μl |  |  | 0.5 μl |  |

After taking out the 24-well plate in which the cells are cultured from the incubator, the culture medium was removed and the plate was washed with 1 × PBS in order to eliminate the remaining culture medium. After further removing 1 × PBS, the transgenic culture medium was dispensed in each well and incubated for 6 hours in an incubator so that the transgenic mixture entered the cells well. After 6 hours, the culture solution containing the transgenic mixture was removed from the incubator, and the culture solution containing serum (RPMI1640, 10% FBS, 1% P/S) was added and further cultured (6 hours/18 hours/42 hours) .

The 24-well plate on which gene introduction and culture have been completed was taken out of the incubator and the culture medium was removed. In order to extract RNA from the cells adhered to the bottom of the plate, TRIzol reagent was used to treat the cells, and then, RNA was extracted according to the method recommended by the vendor.

In order to synthesize cDNA from the extracted RNA, a reagent M-MLV RT 5 × master mix (Elpisbiotech, #EBT-1511) was used, and cDNA was synthesized from RNA according to the method recommended by the vendor.

Real-time PCR analysis was performed to quantitatively analyze the expression of target gene from cDNA. Power SYBR Green PCR Master Mix (#4367659) reagent was used, and the expression level of the target gene from cDNA was confirmed according to the method recommended by the vendor.

### (1) Interferon beta

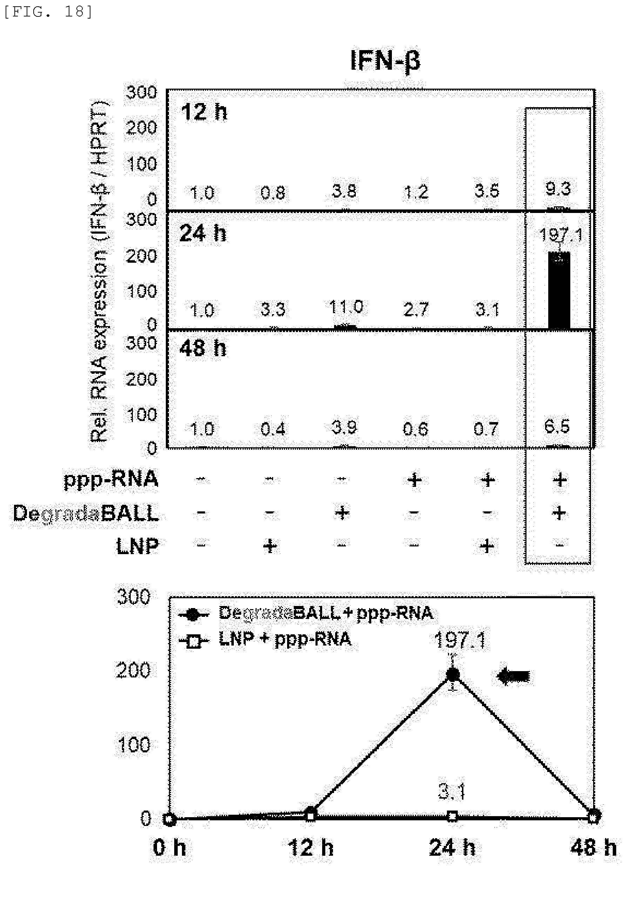

There was little change in IFN-β in the Control, Group 1 and Group 2 without addition of ppp-RNA. This is because LNP and DegradaBALL only as a carrier without ppp-RNA do not affect the expression of IFN-β (FIG. 18).

In the case of the experimental group with addition of ppp-RNA without carrier, there was no change in expression of IFN-β regardless of the incubation time. Therefore, it may be construed as not affecting the expression of IFN-β because the ppp-RNA could not enter the cell even if ppp-RNA was present.

In the case of the experimental group in which ppp-RNA was added and LNP was used as a carrier, there was no significant change in IFN-β expression regardless of the incubation time. Therefore, it may be construed that the ppp-RNA could not be delivered into the cell with LNP alone, or that, even if the ppp-RNA was delivered into the cell by LNP, ppp-RNA would have lost activity from intracellular environment before interaction with RIG-I.

In the case of the experimental group in which ppp-RNA was added and DegradaBALL was used as a carrier, it could be seen that IFN-β expression was significantly changed according to the culture time. In particular, when the incubation time was 24 hours, the highest expression of IFN-β was observed. Therefore, DegradaBALL effectively delivers ppp-RNA into the cells and effectively demonstrates biological activity thereof.

### (2) Viperin

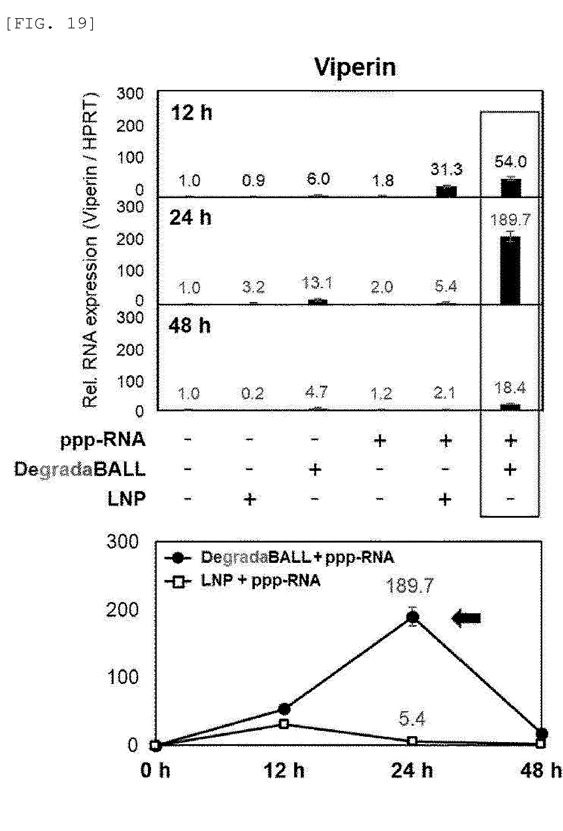

There was little change in Viperin in the Control, and Group 1 without addition of ppp-RNA. Therefore, it may be construed that the interaction with RIG-I does not occur due to no existence of ppp-RNA and that only LNP as a carrier does not affect the expression of Viperin (FIG. 19).

The change in Viperin of Group 2 without addition of ppp-RNA but with addition of DegradaBALL alone was not significant.

In the case of Group 3 with addition of ppp-RNA but no carrier, there was no change in Viperin expression regardless of the incubation time. Therefore, it may be construed that the expression of Viperin is not influenced because ppp-RNA did not enter the cell even if ppp-RNA is present.

In the case of Group 4 in which ppp-RNA was added and LNP was used as a carrier, there was a slight increase in Viperin expression only in a short incubation time of 12 hours. Therefore, it may be construed that biological effects of ppp-RNA by LNP are not great but limited.

In the case of Group 5 where ppp-RNA was added and DegradaBALL was used as a carrier, it was observed that Viperin expression was greatly increased according to incubation time. In particular, at a time of 24 hours after incubation, the highest expression of Viperin was obtained. Therefore, it may be construed that DegradaBALL effectively delivers ppp-RNA into the cells and effectively exhibits biological activity thereof.

**(3) Interferon alpha**

C57BL/6 8-week-old mice were divided into experimental groups summarized as shown in the table 3 below.

[TABLE 3]

|  | Control | Group 1 | Group 2 | Group 3 |
|---|---|---|---|---|
| ppp-RNA | - | 10 μg | - | 10 μg |
| DegradaBALL | - | - | 200 μg | 200 μg |

Each sample was mixed in 1 × PBS to form a composite and left for 30 minutes to prepare an injection solution, and a final volume of the injection solution was adjusted to be 100 μl. Each group of mice was injected intravenously with each injection solution, and the spleen of the mouse was removed 6 hours, 12 hours and 24 hours after the injection. RNA was extracted from the removed spleen using TRIzol.

In order to synthesize cDNA from the extracted RNA, a reagent M-MLV RT 5 × master mix (Elpisbiotech, #EBT-1511) was used, and RNA was prepared. Further, according to the method recommended by the vendor, cDNA was synthesized from RNA.

Real-time PCR analysis was performed to quantitatively analyze the expression of the target gene from cDNA. Power SYBR Green PCR Master Mix (#4367659) reagent was used. Further, according to the method recommended by the vendor, an expression level of the target gene from cDNA was determined.

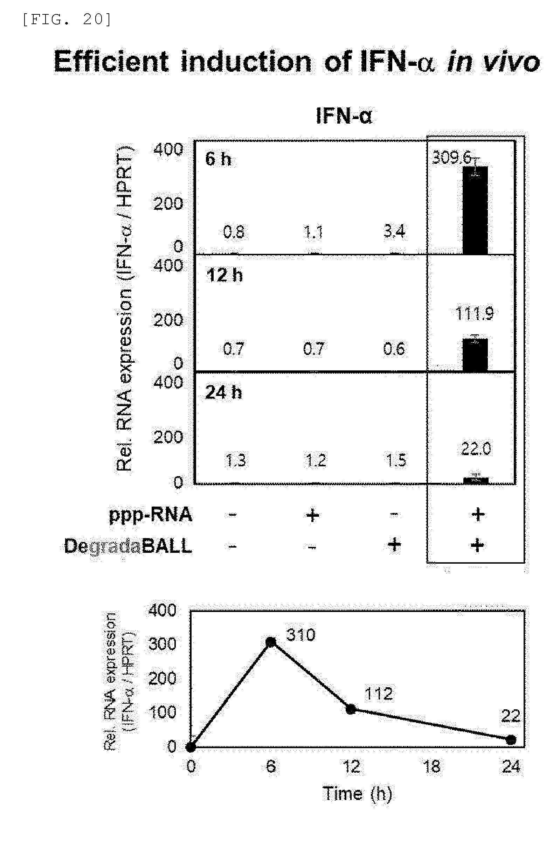

In the cases of Control, Group 1 and Group 2 any change in IFN-α expression could not be observed. In particular, it could be seen that injection of ppp-RNA alone or injection of DegradaBALL alone does not affect the expression of IFN-α (FIG. 20). Further, even when ppp-RNA alone was injected by intravenous injection, this did not invade into the cells. Therefore, the reason of the above fact may be considered that activity of ppp-RNA was quickly lost in the bloodstream.

In the case of Group 3, IFN-α expression is very largely induced, and it could be seen that the expression is continued for up to 24 hours depending on time. Therefore, it may be construed that ppp-RNA loaded on DegradaBALL administered via intravenous injection remains stable in the bloodstream, and ppp-RNA can be delivered into the cells.

**9. Anti-tumor effect**

**(1) Interferon-dependent or non-independent tumor cell death induced by intratumoral LEM-S403 injection (FIGS. 21 to 26)**

In order to determine how long LEM-S403 remains when this is injected into the tumor, an experiment was conducted.

When LEM-S403 is administered by intratumoral injection, it may be expected that OH-RNA is protected by DegradaBALL (vehicle) and slowly released in the tumor. When FITC fluorescence was labeled on OH-RNA and TRAMRA fluorescence was labeled on DegradaBALL, and then OH-RNA was supported and administered by intratumoral injection, the strongest OH-RNA fluorescence was observed on day 1 among day 1, 3 and 5. Further, it could be seen that the above fluorescence gradually decreased over time. On the other hand, in the group administered with OH-RNA alone, fluorescence of OH-RNA could not be confirmed even on day 1. When LEMS-403 is ingested into tumor cells, ppp-RNA is released in a sustained manner. Thereafter, ppp-RNA may increase the secretion of type 1 interferon through a signal mediated by RIG-I, which activates CD 8T cells and NK cells while enhancing immunity. Further, during RIG-I-mediated signaling, a tumor cell apoptosis factor is increased independently of interferon, thereby inducing the death of tumor cells. After subcutaneously injecting $1 \times 10^6$ B16F10 melanoma tumor cells (B16F10) into mice, buffer, vehicle (70 μg), ppp-RNA (7 μg) or LEM-S403 (7 μg) was administered by intratumoral injection on 3 days (day 0) and 5 days (day 2) after injection of the tumor cells, and then, the tumor was isolated on 6 days (day 3) in order to confirm a treatment mechanism. In the tumors isolated through immunofluorescence staining analysis, it was confirmed that the expression of phospho-STAT1 was uniquely increased in the LEM-S403 administration group. Therefore, it is considered that type 1 interferon secreted by LEM-S403 may respond to receptors around or in the cells, thereby inducing phosphorylation of STAT1. Through hematoxylin and eosin staining, it was confirmed that tumor tissues of the LEM-S403 administration group have a smaller number of cells and looser tissue than other treatment groups in histopathological aspects. Further, in TUNNEL analysis through immunofluorescence staining, many more fluorescent portions were observed than other groups. From the above results, it could be confirmed that LEM-S403 caused apoptosis of tumor cells.

**(2) Induction of interferon-independent tumor cell apoptosis due to intratumoral LEM-S403 injection (FIGS. 27 to 31)**

A melanoma mouse model was prepared by subcutaneous injection of $1 \times 10^6$ B16F10 cells into C57BL/6 mice. When the tumor reached a size of about 100 mm$^3$, 6 mice in each group were treated by injecting 50 $\mu$l $1 \times$ PBS (buffer), 70 $\mu$g DegradaBALL (vehicle), 7 $\mu$g ppp-RNA, or 70 $\mu$g DegradaBALL + 7 $\mu$g ppp-RNA (LEM-S403) intratumorally twice at an interval of 2 days. 24 hours after the last administration, the tumors extracted by sacrificing mice were stained with Annexin V-propidium iodide (PI), followed by flow cytometric analysis. As compared to the buffer, vehicle and ppp-RNA treatment groups, it was confirmed in the LEM-S403 treatment group that the proportion of cells under apoptosis (Annexin V+, PI-) and dead cells (Annexin V+, PI+) in the tumor are all significantly increased. Further, it was confirmed that the proportion of living cells (Annexin V-, PI-) was significantly decreased. At the molecular level, it was confirmed that LEM-S403 treatment has increased cleavage of procaspase-3 and expression of the pro-apoptotic gene, that is, Noxa, through western blot and RT-PCR, respectively. These phenomena were also reproduced in *in vitro* experiments. Specifically, B16F10 cells were treated with buffer, 0.85 $\mu$g ppp-RNA, 8.5 $\mu$g DegradaBALL (vehicle), ppp-RNA 0.85 $\mu$g + DegradaBALL 8.5 $\mu$g (LEM-S403) or ppp-RNA 0.85 $\mu$g + Lipofectamine 2000, respectively, and then collected 24 hours after treatment. As a result of RT-PCR analysis of the collected cells, it was confirmed that the expression of Noxa was increased when the cells were treated with LEM-S403. Therefore, it could be verified that the intratumoral injection of LED-S403 may increase expression of Noxa in tumor cells and induce apoptosis of tumor cells according to a mechanism of inducing the cleavage of caspase-3.

**(3) Assessment of increase in the number and activity of tumor-infiltrating NK cells and CD8+ T cells due to intratumoral LEM-S403 injection (FIGS. 32 to 35)**

Flow cytometry and immunofluorescence staining analysis were performed to determine whether the invasion of activated immune cells or immune cells into the tumor is increased due to intratumoral LEM-S403 injection. Based on flow cytometry, the distribution of NK cells among the infiltrated immune cells in the tumor in the LEM-S403 administration group was 10.51%, which is increased by 67% compared to the vehicle group, and among them, NK cells expressing CD 69 were about 3.59% of the infiltrated immune cells in the tumor, indicating that it was increased by about 152% compared to the vehicle group. Further, the distribution of CD 8T cells among the infiltrated immune cells in the tumor in the LEM-S403 administration group was 8.57%, which is also increased by 128% compared to other vehicle groups. Among them, CD 8T cells expressing CD 69 were 6.54%, indicating that it was increased by about 141% compared to other vehicle groups. Immunofluorescence staining analysis also demonstrated that NK cells and CD 8T cells have infiltrated into the tumor in the LEM-S403 administration group more than the other administration groups, while showing higher proportion of CD 69 positive cells.

**(4) Anti-tumor effects of administration of LEM-S403 alone and in combination (+ anti-PD-1 antibody) in melanoma mouse model (FIGS. 36 to 39)**

In the mouse melanoma model, an experiment was conducted to determine effects of inhibiting tumor growth by administering LEM-S403 alone or in combination with anti-PD-1 antibody. Specifically, when a volume reached about 100 mm$^3$ after inoculating mice with melanoma cells B16F10, buffer ($1 \times$ PBS, 50 $\mu$L, intratumoral administration), vehicle (70 $\mu$g, intratumoral administration), ppp-RNA (7 $\mu$g, intratumoral administration), LEM-S403 (7 $\mu$g, intratumoral administration) or LEM-S403 (7 $\mu$g, intratumoral administration) and anti-PD-1 antibody (10 mg/kg, intraperitoneal administration) were administered, respectively, followed by measuring the tumor volume at an interval of 2 and 3 days. In the buffer, vehicle, and ppp-RNA administration groups, respectively, tumors grew rapidly. On the other hand, in the administration groups of LEM-S403 alone and in combination with anti-PD-1 antibody, tumor growth was increased slowly from day 2. Since day 7, it was confirmed that the tumor volume was significantly small as compared to the vehicle group. Further, after completing the administration, it was confirmed that the tumor growth was significantly suppressed compared to the vehicle group even on day 11. Survival rates of all mice were followed up, and all mice belonging to the buffer, vehicle and ppp-RNA administration groups died before 15 days. However, in the group administered with LEM-S403 alone or in combination with anti-PD-1 antibody, the average survival time was significantly increased. Further, when administered in combination with anti-PD-1, no significant difference in tumor volume was observed compared to administration of LEM-S403 alone. However, it was confirmed that the mean survival time was significantly increased.

**10. Confirmation of intracellular delivery according to the charge of RNA-carrying particles**

The synthetic RIG-I ligand of SEQ ID NO: 1 used in Example 7 (5'-triphosphate hairpin RNA, ppp-RNA) and the porous silica particles of Example 1-1-(11) were mixed by concentration and delivered to B16F10 cells.

Each well was inoculated with $5 \times 10^4$ cells, followed by delivering the particles under the conditions of 4 h Transfection (Serum free) + 2 h (10% FBS).

The charge of each particle is shown in Table 4 below.

[TABLE 4]

| particle ($\mu$g/ml) | pppRNA($\mu$g/ml) | carrier particle charge |
|---|---|---|
| 10 | 1 | 28.8 |
| 10 | 0.1 | 39.8 |
| 10 | 0 | 41.5 |

Referring to FIG. 41, it can be seen that, when cells were treated with particles and ppp-RNA in a ratio of 10:0.1, temporary vacuoles (vacuoles) were formed in the cells. However, at the ratio of 10:1 (particle:ppp-RNA), formation of vacuoles was not observed.

## Claims

1. A pharmaceutical composition for improving immune activity, comprising:

   a blunt-ended hairpin RNA represented by Formula (1) below; and
   porous silica particles carrying the RNA in pores thereof,
   wherein the porous silica particles have an average pore diameter of 7 to 25 nm, and the inside of the pores is positively charged,

   [Formula 1]        5'-$P_a$-((N1-X1)$_b$-N2)-UUCG-(N3-(X2-N4)$_b$)-3'

   (wherein P is a phosphoric acid group,
   a is an integer of 2 to 5, and b is an integer of 1 to 5,
   UUCG is a base to form a loop of the hairpin,
   N1 and N2 are 2 to 4 bases selected from G or C, X1 and X2 are 1 to 5 bases selected from A or U, and the plurality of selected bases are the same as or different from one other,
   N3 is complementarily bound with N2, X2 is complementarily bound with X1, and N4 is complementarily bound with N1, and
   each base repeating b times is the same as or different from one another).

2. The composition according to claim 1, wherein the RNA is represented by Formula 2 below:

   [Formula 2]        5'-$P_a$-((N1-X1)$_b$-N2)-UUCG-(N3-(X2-N4)$_b$)-3'

   (wherein P is a phosphoric acid group,

   a and b are each an integer of 2 to 4,
   N1 and N2 are 2 to 4 bases selected from G or C, X1 and X2 are 2 to 4 bases selected from A or U, and the plurality of selected bases are the same as or different from one other,
   N3 is complementarily bound with N2, X2 is complementarily bound with X1, and N4 is complementarily bound with N1, and

each base repeating b times is the same as or different from one another).

3. The composition according to claim 1, wherein the RNA has a length of 14 to 100 nt.

4. The composition according to claim 1, wherein the RNA includes any one of SEQ ID NOs: 1 to 25, as well as 2 to 4 phosphoric acid groups bound to the 5'-terminal.

5. The composition according to claim 1, wherein a zeta potential of the porous silica particles carrying RNA in the pores thereof is 5 to 65 mV.

6. The composition according to claim 1, wherein the zeta potential of the porous silica particles carrying the RNA in the pores thereof is 35 mV or less.

7. The composition according to claim 1, wherein the zeta potential of the particle without carrying RNA is 10 to 70 mV.

8. The composition according to claim 1, wherein a weight ratio of the particles and the RNA is 1: 5 to 20.

9. The composition according to claim 1, wherein the particle has a plurality of pores, and the pores extend from a surface to an inside of the particle.

10. The composition according to claim 1, wherein a BET surface area of the particles is 280 to 680 $m^2/g$, and a particle diameter is 50 to 500 nm.

11. The composition according to claim 1, wherein the porous silica particles have pores of small pore particles having an average pore diameter of less than 5 nm, and the pores are expanded to an average diameter of 7 to 25 nm.

12. A pharmaceutical composition for prevention or treatment of cancer, comprising:

   a blunt-ended hairpin RNA represented by Formula (1) below; and
   porous silica particles carrying the RNA in pores thereof,
   wherein the porous silica particles have an average pore diameter of 7 to 25 nm, and the inside of the pores is positively charged,

   [Formula 1]     5'-$P_a$-((N1-X1)$_b$-N2)-UUCG-(N3-(X2-N4)$_b$)-3'

   (wherein P is a phosphoric acid group,
   a is an integer of 2 to 5, and b is an integer of 1 to 5,
   UUCG is a base to form a loop of the hairpin,
   N1 and N2 are 2 to 4 bases selected from G or C, X1 and X2 are 1 to 5 bases selected from A or U, and the plurality of selected bases are the same as or different from one other,
   N3 is complementarily bound with N2, X2 is complementarily bound with X1, and N4 is complementarily bound with N1, and
   each base repeating b times is the same as or different from one another).

13. The composition according to claim 12, wherein the RNA is represented by Formula 2 below:

   [Formula 2]     5'-$P_a$-((N1-X1)$_b$-N2)-UUCG-(N3-(X2-N4)$_b$)-3'

   (wherein P is a phosphoric acid group,

   a and b are each an integer of 2 to 4,
   N1 and N2 are 2 to 4 bases selected from G or C, X1 and X2 are 2 to 4 bases selected from A or U, and the plurality of selected bases are the same as or different from one other,
   N3 is complementarily bound with N2, X2 is complementarily bound with X1, and N4 is complementarily bound with N1, and
   each base repeating b times is the same as or different from one another)

14. The composition according to claim 12, wherein the RNA has a length of 15 to 100 nt.

15. The composition according to claim 12, wherein RNA includes any one sequence of SEQ ID NOs: 1 to 25, and 2 to 4 phosphoric acid groups bound to the 5-terminal.

16. The composition according to claim 12, wherein a zeta potential of the porous silica particles carrying the RNA in the pores thereof is 5 to 65 mV.

17. The composition according to claim 12, wherein the zeta potential of the porous silica particles carrying the RNA in the pores thereof is 35 mV or less.

18. The composition according to claim 12, wherein the zeta potential of the particle without carrying RNA is 10 to 70 mV.

19. The composition according to claim 12, wherein a weight ratio of the particles and the RNA is 1: 5 to 20.

20. The composition according to claim 12, wherein the particle has a plurality of pores, and the pores extend from a surface to an inside of the particle.

21. The composition according to claim 12, wherein a BET surface area of the particle is 280 to 680 $m^2/g$, and a particle diameter is 50 to 500 nm.

22. The composition according to claim 12, wherein the porous silica particles have pores of small pore particles having an average pore diameter of less than 5 nm, and the pores are expanded to an average diameter of 7 to 25 nm.

23. The composition according to claim 12, wherein the cancer is any one of breast cancer, ovarian cancer, cervical cancer, prostate cancer, testicular cancer, penile cancer, genitourinary tract cancer, testicular tumor, esophageal cancer, laryngeal cancer, gastric cancer, gastrointestinal cancer, skin cancer, keratinocyte cell tumor, follicular carcinoma, melanoma, lung cancer, small cell lung carcinoma, non-small cell lung carcinoma (NSCLC), lung adenocarcinoma, squamous cell carcinoma of the lung, colon cancer, pancreatic cancer, thyroid cancer, papillary cancer, bladder cancer, liver cancer, bile duct cancer, kidney cancer, bone cancer, bone marrow disorder, lymphatic disorder, hair cell cancer, oral and pharyngeal (oral) cancer, lip cancer, tongue cancer, oral cancer, salivary gland cancer, pharyngeal cancer, small intestine cancer, colon cancer, rectal cancer, kidney cancer, prostate cancer, vulvar cancer, thyroid cancer, colorectal cancer, endometrial cancer, uterine cancer, brain cancer, central nervous system cancer, peritoneal cancer, hepatocellular carcinoma, head cancer, neck cancer, Hodgkin disease and leukemia.

[FIG. 1]

[FIG. 2]

[FIG. 3]

[FIG. 4]

2 L scale  10 L scale

200 nm  200 nm

[FIG. 5]

2nm  4nm  7nm  10nm  23nm

200 nm

50 nm

[FIG. 6]

0 hr          120 hrs          360 hrs

[FIG. 7]

DDV solution

Dialysis membrane

Fresh SBF

[FIG. 8]

$t(DC_{50}) = \sim 2.4$ days ($\sim 58$ hr) ($\pm 20\%$)
$t(DC_{80}) = \sim 3.9$ days ($\sim 93$ hr) ($\pm 20\%$)
$t(DC_{95}) = \sim 7$ days ($\sim 168$ hr) ($\pm 20\%$)

$r^2 (t(DC_0) \sim t(DC_{80})) = 0.97$

[FIG. 9]

| Sample | $t_{50\%}$ |
|---|---|
| DDV(200)$_{10}$ | 57.4 h |
| DDV(500)$_{10}$ | 37.5 h |
| DDV(1000)$_{10}$ | 30.0 h |

[FIG. 10]

| Samples | $t_{50\%}$ |
|---|---|
| MSN(200)$_2$ | 17.9 h |
| DDV(200)$_{10}$ | 57.4 h |
| DDV(200)$_{17}$ | 53.6 h |

[FIG. 11]

## DDV(300)$_{17}$

[FIG. 12]

## DDV(300)$_{17}$−NH$_2$

$t_{50\%}$ = 약 2.5 days

[FIG. 13]

**A**

100 nm

[FIG. 14]

**B**

ppp-RNA

5' PPP-GGAUCGAUCGAUCG U U
         |||||||||||||
3'OH-CCUAGCUAGCUAGC G C

OH-RNA

5' OH-GGAUCGAUCGAUCG U U
        |||||||||||||
3'OH-CCUAGCUAGCUAGC G C

[FIG. 15]

**C**

| ratio | Ctrl | 1:40 | 1:30 | 1:20 | 1:10 | 1:5 |
|---|---|---|---|---|---|---|
| ppp-RNA | 1 | 1 | 1 | 1 | 1 | 1 |
| DeradaBALL | 0 | 40 | 30 | 20 | 10 | 5 |

Hairpin RNA

[FIG. 16]

**D**

[FIG. 17]

**E**

[FIG. 18]

[FIG. 19]

**Viperin**

[FIG. 20]

# Efficient induction of IFN-α *in vivo*

[FIG. 21]

[FIG. 22]

[FIG. 23]

**Type 1 IFNs**
- Increased cytotoxicity of NK cells and CD8 T cells
- Increased T cell cross priming by Dendritic cells
- Pro-inflammatory cytokine modulation

**RIG-I induced apoptosis**
- RIG-I induces apoptotic cell death in tumor cells
- Increased apoptotic gene expression and caspase cleavage

[FIG. 24]

**D**

C57/B6 mouse (5w, male)

Day 0          2     3

1x10⁶ B16F10 cells
subcutaneous injection

Treatment
(Buffer/Vehicle/ppp-RNA/LEM-S403)

✓Sacrifice & Analysis

[FIG. 25]

**E**

|  | Buffer | Vehicle | ppp-RNA | LEM-S403 |
| Merged | | | | |
| Phospho-STAT1 | | | | |
| Tumor cells (DAPI) | | | | |

Scale bar: 100 μm

[FIG. 26]

Scale bar: 100 μm

[FIG. 27]

Scale bar: 100 μm

[FIG. 28]

[FIG. 29]

[FIG. 30]

[FIG. 31]

# E

B16F10 (*in vivo*)

[FIG. 32]

[FIG. 33]

Scale bar: 100 µm

[FIG. 34]

EP 3 936 118 A1

[FIG. 35]

Scale bar: 100 μm

[FIG. 36]

[FIG. 37]

[FIG. 38]

[FIG. 39]

**D**

[FIG. 40]

[FIG. 41]

| nanoparticle | 0 | 0 | 10 | 10 | ug/ml |
| ppp-RNA | 0 | 1 | 0.1 | 1 | ug/ml |

[FIG. 42]

## INTERNATIONAL SEARCH REPORT

| International application No. |
| --- |
| PCT/KR2020/002648 |

### A. CLASSIFICATION OF SUBJECT MATTER

*A61K 9/14(2006.01)i, A61K 9/16(2006.01)i, A61K 31/7105(2006.01)i, A61P 37/00(2006.01)i, A61P 35/00(2006.01)i*

According to International Patent Classification (IPC) or to both national classification and IPC

### B. FIELDS SEARCHED

Minimum documentation searched (classification system followed by classification symbols)

A61K 9/14; A61K 31/695; A61K 47/42; A61K 47/48; A61K 9/127; A61K 9/24; A61K 9/51; A61P 31/00; C12N 9/22; A61K 9/16; A61K 31/7105; A61P 37/00; A61P 35/00

Documentation searched other than minimum documentation to the extent that such documents are included in the fields searched
Korean utility models and applications for utility models: IPC as above
Japanese utility models and applications for utility models: IPC as above

Electronic data base consulted during the international search (name of data base and, where practicable, search terms used)
eKOMPASS (KIPO internal) & Keywords: immune, antibody, RNA, porous silica, carrier

### C. DOCUMENTS CONSIDERED TO BE RELEVANT

| Category* | Citation of document, with indication, where appropriate, of the relevant passages | Relevant to claim No. |
| --- | --- | --- |
| A | KR 10-2014-0103914 A (STC.UNM et al.) 27 August 2014<br>See claims 1-38; figure 2. | 1-23 |
| A | KR 10-2014-0030420 A (KOREA UNIVERSITY RESEARCH AND BUSINESS FOUNDATION) 12 March 2014<br>See the entire document. | 1-23 |
| A | WO 2017-181115 A1 (SPINNAKER BIOSCIENCES, INC. et al.) 19 October 2017<br>See the entire document. | 1-23 |
| A | US 2018-0049984 A1 (BRINKER, C. J. et al.) 22 February 2018<br>See the entire document. | 1-23 |
| A | WO 2017-041033 A1 (BRINKER, C. J. et al.) 09 March 2017<br>See the entire document. | 1-23 |

| ☐ Further documents are listed in the continuation of Box C. | ☒ See patent family annex. |
| --- | --- |

| * Special categories of cited documents: | "T" later document published after the international filing date or priority date and not in conflict with the application but cited to understand the principle or theory underlying the invention |
| --- | --- |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| --- | --- |
| 11 JUNE 2020 (11.06.2020) | **11 JUNE 2020 (11.06.2020)** |

| Name and mailing address of the ISA/KR<br>Korean Intellectual Property Office<br>Government Complex Daejeon Building 4, 189, Cheongsa-ro, Seo-gu,<br>Daejeon, 35208, Republic of Korea<br>Facsimile No. +82-42-481-8578 | Authorized officer<br><br>Telephone No. |
| --- | --- |

Form PCT/ISA/210 (second sheet) (January 2015)

**INTERNATIONAL SEARCH REPORT**
Information on patent family members

International application No.

**PCT/KR2020/002648**

| Patent document cited in search report | Publication date | Patent family member | Publication date |
|---|---|---|---|
| KR 10-2014-0103914 A | 27/08/2014 | AU 2014-323937 A1 | 05/06/2014 |
| | | CA 2852064 A1 | 18/04/2013 |
| | | CN 103396164 A | 20/11/2013 |
| | | CN 103396164 B | 08/10/2014 |
| | | CN 104023711 A | 03/09/2014 |
| | | EP 2765997 A2 | 20/08/2014 |
| | | JP 2014-532071 A | 04/12/2014 |
| | | MX 2014004415 A | 05/06/2015 |
| | | US 2015-0272885 A1 | 01/10/2015 |
| | | US 2017-0232115 A1 | 17/08/2017 |
| | | WO 2013-056132 A2 | 18/04/2013 |
| | | WO 2013-056132 A3 | 13/06/2013 |
| KR 10-2014-0030420 A | 12/03/2014 | KR 10-1975754 B1 | 09/05/2019 |
| WO 2017-181115 A1 | 19/10/2017 | AU 2018-250300 A1 | 01/11/2018 |
| | | CA 3021001 A1 | 19/10/2017 |
| | | CN 109843301 A | 04/06/2019 |
| | | EP 3442540 A1 | 20/02/2019 |
| | | JP 2019-511582 A | 25/04/2019 |
| US 2018-0049984 A1 | 22/02/2018 | US 2016-0090603 A1 | 31/03/2016 |
| | | US 2018-0028686 A1 | 01/02/2018 |
| | | WO 2016-054225 A1 | 07/04/2016 |
| WO 2017-041033 A1 | 09/03/2017 | US 2018-0344641 A1 | 06/12/2018 |

Form PCT/ISA/210 (patent family annex) (January 2015)

**EP 3 936 118 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2014039961 A, Stone